(19) Europäisches Patentamt
European Patent Office
Office européen des brevets

(11) Publication number: **0 585 999 A1**

# (12) EUROPEAN PATENT APPLICATION

(21) Application number: **93202330.2**

(22) Date of filing: **07.08.93**

(51) Int. Cl.[5]: **C07D 495/14**, C07D 333/54, C07D 333/76, C07D 307/91, C07D 495/04, G02F 1/35

(30) Priority: **14.08.92 US 930732**

(43) Date of publication of application: **09.03.94 Bulletin 94/10**

(84) Designated Contracting States: **AT BE CH DE DK ES FR GB GR IE IT LI LU MC NL PT SE**

(71) Applicant: **ENICHEM S.p.A.**
**Piazza della Repubblica, 16**
**I-20124 Milano(IT)**

(72) Inventor: **Jen, Kwang-Yue Alex**
**8 Schindler Drive North**
**Old Bridge, New Jersey 08857(US)**
Inventor: **Varanasi, Pushkara Rao**
**1207 Sweetbriar Street**
**Monmouth Junction, New Jersey 08536(US)**
Inventor: **Drost, Kevin Joel**
**23 Chestnut Street**
**Spotwood 08884 New Jersey(US)**
Inventor: **Wong, King Young**
**6010 Shadow Oaks Court**
**Monmouth Junction, New Jersey 08852(US)**

(74) Representative: **De Carli, Erberto**
**ING. BARZANO' & ZANARDO MILANO S.p.A.**
**Via Borgonuovo, 10**
**I-20121 Milano (IT)**

## (54) Functional heteroaromatics for NLO applications.

(57) Nonlinear optical materials having highly conjugated fused ring structures of two or three aromatic or heteroaromatic rings, at least one of which is a five-membered heteroaromatic ring, or structures of one to four non-fused five-membered heteroaromatic rings. Methods of tricyanovinylating heteroaromatic ring structures to form nonlinear optical materials are also disclosed. Polymers having the disclosed nonlinear optical materials as pendant side chains, which polymers exhibit second order nonlinear optical properties, and base polymers having pendant fused ring structures devoid of nonlinear optical properties that exhibit second order nonlinear optical properties after the covalent attachment of tricyanovinyl groups to the pendant side chains are disclosed. Methods of tricyanovinylating the pendant side chains of the base polymers are also disclosed.

EP 0 585 999 A1

## BACKGROUND OF THE INVENTION

### FIELD OF THE INVENTION

The present invention relates to heteroaromatic compounds with nonlinear optical (NLO) properties. In particular, the present invention relates to NLO materials having highly conjugated fused ring structures of two or three aromatic rings, at least one of which is a five-membered heteroaromatic ring. The present invention also relates to NLO materials having highly conjugated structures of one to four non-fused five-membered heteroaromatic rings.

The compounds of the present invention, once suitably oriented, are capable of highly efficient second harmonic generation and electro-optic modulation of an electromagnetic wave having a wave length between 300 nm and 2,000 nm. The present invention further relates to the incorporation of the compounds of the present invention into polymeric matrices, including polymers having side chains of the disclosed compounds.

### DESCRIPTION OF THE PRIOR ART

Highly efficient NLO materials capable of doubling or tripling the frequency of incident light are currently of great scientific and technological interest for use in optical telecommunications, signal processing and the construction of optical computers. Nonlinear optics is concerned with the interaction of electromagnetic fields in various media to produce new fields which may be altered in phase, frequency or amplitude. The NLO effect of a material upon an electromagnetic field is a function of the second and higher order terms of the following equation:

$$P = \alpha E + \beta E^2 + \gamma E = ^3 + . . .$$

P is the polarization of a material, E is the intensity of the electric field, and the coefficients $\alpha$, $\beta$, $\gamma$, etc. are indicative of the NLO susceptibility of the material. Such coefficients are constant for a given material, but vary from material to material. The second order coefficient, $\beta$, for a given material, is indicative of the second harmonic generation properties of the material, with second harmonic generation efficiencies increasing as the value of $\beta$ increases.

Candidate NLO materials should possess good physical properties, such as high optical transparency, low dielectric constant, high laser damage threshold and good solubility in the solvents used for spin-casting of optical materials, and the like. The materials should also possess the mechanical and thermal properties required of optical materials, in particular, high $\beta$ values, fast response times and nonlinear susceptibility over a broad range of wavelengths, particularly at wavelengths between about 300 nm and about 2,000 nm.

The first NLO materials were monocrystal minerals such as $KH_2PO_4$, $LiNbO_3$, InSb and $NH_4H_2PO_4$. However, these materials were costly to grow in high optical quality, showed relatively low second harmonic generating properties, and have been replaced by organic and polymeric materials with large delocalized pi-electron systems. The organic and polymeric materials with large delocalized pi-electron systems exhibit greater nonlinear susceptibilities and can be varied to optimize the desired physical and mechanical properties.

Early organic NLO materials were based upon conjugated pi-electron chromophores with charge asymmetry such as 4-dimethylamino-4-nitrostilbene. This material is disclosed by Williams, Angew. Chem. Int. Ed. Engl., 23, 690-703 (1984). However, such combinations were of limited solubility, resulting in crystallization of the guest chromophore molecule out of the host matrix, or mobility of the guest molecules in the matrix, resulting in a loss of second harmonic generating performance. Such materials are also exemplified by U.S. Patent No. 4,892,681 to Miyata and U.S. Patent No. 4,894,186 to Gordon.

The insolubility of these materials in a polymer matrix was overcome by the covalent linking of the NLO chromophores to the polymer backbones. This is also disclosed by Williams, as well as U.S. Patent Nos. 4,894,263 to Dubois, 4,933,112 to DeMartino and 4,935,292 to Marks. These references disclose polymers having NLO chromophore side chains of a series of aromatic rings separated by pi-electron conjugated carbon-carbon, carbon-nitrogen and nitrogen-nitrogen bridges. The aromatic rings disclosed are based on six-membered rings such as benzene and pyridine.

Some azomethine-derived chromophores, which contain five-membered heteroaromatic rings, are disclosed as having third order NLO susceptibilities by Dirk, Proc. SPIE-INT. Soc. Opt. Eng., 1147, 18-25 (1989). However, the reported third order susceptibility of these materials is low, and second order

properties cannot be reasonably predicted from third order susceptibilities, let alone from low-value third order susceptibility.

Methods by which polymers having chromophore side chains may be prepared vary. U.S. Patent No. 4,933,112 discloses the attachment of the chromophore to a monomer that is then polymerized. U.S. Patent No. 4,935,292 discloses the attachment of the chromophore to a functionalized polymer. U.S. Patent No. 4,894,263 discloses that either method of attachment may be used depending on the constituents of the material. It is further disclosed that the constituents of the chromophore may be assembled in one or more steps.

Once polymerized, the polymer is spin-cast to form a film, which is then heated to near it glass-rubber transition temperature ($T_g$) to enhance molecular motion, including rotation of the chromophore side chains. An intense electric field is then applied to the heated film for a given length of time and the film is then cooled to well below the $T_g$ in the presence of the electric field. This results in alignment of the dipoles of the side chains, providing a system in which the NLO components are locked in a preferred orientation while at the same time covalently linked within a polymer matrix. According to U.S. Patent No. 4,935,292, NLO efficiency can be increased by repeatedly heating the material above and then cooling it below the $T_g$ several times prior to applying the electric field. It is disclosed that this reduces the number of pinholes, voids, free volume and other anomalies that can cause short circuits during poling, and also removes residual stress from the film.

The pi-electron conjugated carbon-carbon, carbon-nitrogen and nitrogen-nitrogen bridges linking the aromatic or heteroaromatic rings of the NLO chromophores to the prior art are thermally or photochemically unstable, or both, under the above conditions. Higher temperatures cause isomerization of the stilbene, diene and azo-type pi-bridges. Trans isomers are converted to cis isomers, which possess much lower NLO activity. Azomethine-type linkages are decomposed by elevated temperatures. Acetylene-type bridges are less effective in enhancing NLO activity and are reactive to many common reagents. A need exists for NLO materials in which the thermal and photochemical stability properties have been improved without a sacrifice of NLO properties.

Eliminating the pi-conjugated bridges between aromatic and heteroaromatic rings would benefit the thermal and photochemical stability of the prior art conjugated compounds. The cis-trans isomerization associated with pi-bridges at elevated temperatures or when exposed to UV or visible light would be avoided. CA 2,010,528 discloses an NLO compound based on thiophene rings linked by non-conjugated bridges. EP 353,606 discloses nonlinear optical compounds based on a single imidazole ring. JP 3-005,732 discloses NLO materials of the formula:

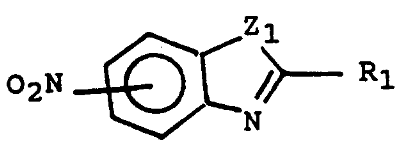

and

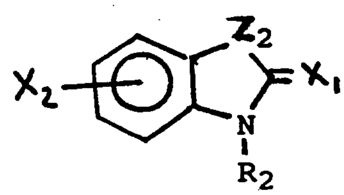

in which Z can be oxygen or sulfur. These prior art materials provide improvements in thermal and photochemical stability at a sacrifice of NLO properties.

JP 1-101,522, JP 1-101,523, U.S. Patent No. 3,978,029, Tazuke et al., Makromol. Chem., 181, 2199-2206 (1980) and Oshiro et al., Polym. J., 6(5), 364-9 (1974) disclose carbazole and indole compounds having NLO properties. These compounds likewise provide an improvement in thermal and photochemical stability at a sacrifice of NLO properties.

The need for an improvement in the thermal and photochemical stability of NLO materials without a loss of NLO properties has not been satisfied to date.

## SUMMARY OF THE INVENTION

Heteroaromatic compounds have been discovered that are thermally and photochemically stable and possess advantageous NLO properties such as high second order susceptibilities, good solubility and ease of functionalization. The compounds contain two to four aromatic or heteroaromatic rings, at least one of which is a five-membered heteroaromatic ring. The use of heteroaromatics maintains an effective conjugated chain length while eliminating pi-conjugated bridges between aromatic rings. This maintains optical nonlinearity while benefiting thermal and photochemical stability. The introduction of heteroaromatic rings

also improves solubility and selectivity in functionalization when compared to benzenoid counterparts.

In accordance with the present invention, there is provided a nonlinear optical compound corresponding to Formula I:

D-R-A (I)

wherein R is selected from fused ring systems consisting of two or three aromatic or heteroaromatic rings, at least one of which is a five-membered heteroaromatic ring having a structure corresponding to Formula II:

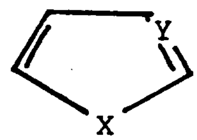

(II)

wherein Y is C or N and X is selected from O, S, Se and N, provided

a) that when R consists of two rings, one of which is benzene, Y is C; and

b) that X is selected from O, S and Se when the ring system has a structure corresponding to Formula III:

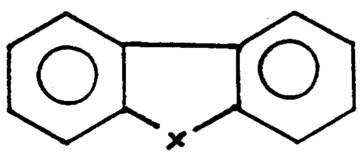

(III)

D is a first electron donating group, A is a first electron accepting group, and D and A are attached to ring members of different rings so that D, A and R form a delocalized resonance configuration.

In accordance with one aspect of the present invention there is provided a nonlinear optical compound corresponding to Formula I, wherein R is a fused ring system consisting of two or three five-membered heteroaromatic rings. In accordance with another aspect of the present invention, there is also provided a nonlinear optical compound corresponding to Formula I wherein R is a fused ring system consisting of one five-membered heteroaromatic ring and one benzene or pyridine ring.

In accordance with a third aspect of the present invention, there is also provided a nonlinear optical compound corresponding to Formula I wherein R is a fused ring system consisting of one five-membered heteroaromatic ring and two rings independently selected from benzene and pyridine rings. In accordance with a fourth aspect of the present invention, there is also provided a nonlinear optical compound corresponding to Formula I wherein R is a fused ring system consisting of two five-membered heteroaromatic rings and a benzene or pyridine ring.

The present invention also incorporates the discovery that one to four non-fused five-membered heteroaromatic rings functionalized with a strong electron donating group, a strong electron accepting group, or both, possess a desirable combination of thermal and photochemical stability and advantageous NLO properties such as high second order susceptibilities and good solubility. Therefore, in accordance with a fifth aspect of the present invention, this is also provided a fifth nonlinear optical compound corresponding to Formula I wherein R is selected from heteroaromatic ring compounds represented by the structure of Formula IIa:

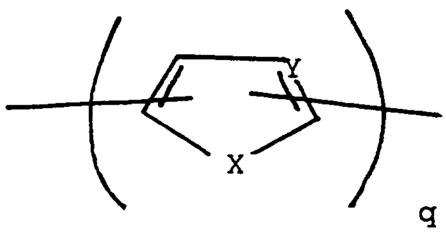

(IIa)

wherein x and y are the same as described above with respect to Formula II and q is an integer from one to four, inclusive. D is an electron donating group, A is an electron accepting group and D, A and the one to four rings of R form a delocalized resonance configuration.

Either D is a strong electron donating group or A is a strong electron accepting group or both D and A are strong electron donating and electron accepting groups, respectively. Strong electron donating groups are selected from $-N(CH_3)_2$, pyrrolidine, dithiolium, dithiane, piperidine, piperazine, morpholine,

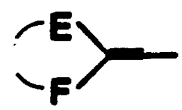 and 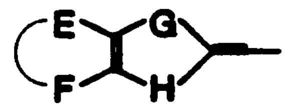

wherein E, F, G and H are members of a saturated or unsaturated five- to eight-membered cyclic ring or two-ring system having five- to eight- membered rings that are electron donating in nature and E, F, G and H are heteroatoms independently selected from O, N, S, Se and Te. Strong electron accepting groups are selected from tricyanoethylene, dicyanoethylene, $-NO_2$, dinitroethylene, nitroesterethylene, nitrocyanoethylene, N,N-dialkylbarbituric acids, N,N-dialkylthiobarbituric acids, 3-dicyanovinylindane-1-sulfone, 1,3-bissulfonylindane, indane-1,3-dione, 3-dicyanoindane-1-one, 1,3-bisdicyanovinylindane and

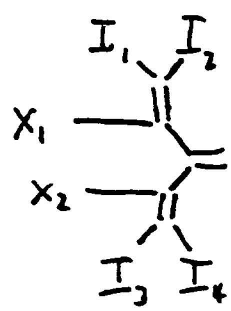

wherein $I_1$, $I_2$, $I_3$ and $I_4$ are independently selected from cyano, nitro, ester, sulfonyl and phosphonyl groups, and $X_1$ and $X_2$ form a saturated or unsaturated five- to eight-membered cyclic ring or two-ring system having five- to eight-membered rings.

One aspect of the present invention provides non-centrosymmetric crystals of the above-mentioned compounds for use as second order NLO materials.

Another aspect of the present invention provides a combination exhibiting second order NLO properties. This combination includes a chemically inert medium and the NLO compounds of the present invention. The NLO compounds of these combinations preferably have an external field-induced molecular alignment.

In one embodiment of this aspect of the invention, the NLO compound is disposed as a layer or layers on a substrate of the chemically inert medium, which chemically inert medium is selected from glass, silica, silicon and polymeric materials. In another embodiment of this aspect of the invention, the NLO compound is in the form of a blend of a guest compound in a host matrix, with the NLO compound of the present invention serving as the guest compound and the chemically inert medium serving as the host matrix, and preferably being a thermoplastic polymer selected from polyacrylates, polymethacrylates, polyacrylamides, polycarbonates, polyamides, polyesters, polystyrenes, polyimides, polyether ketones, polyether etherketones, polyphenylene ethers and copolymers thereof.

In still another embodiment of this aspect of the present invention, pendant side chains of the NLO compounds of the present invention are covalently bonded to a chemically inert polymeric material so that a combination is provided corresponding to Formula IV:

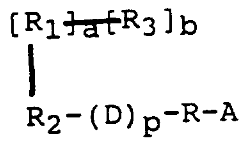

(IV)

wherein $R_1$ and $R_3$ are monomeric subunits independently selected from polyacrylate, polyimide, polyamide, polyacrylamide, polystyrene, polyvinyl halide, polyacrylonitrile, polyvinyl alcohol, polyvinyl acetate, polyester, polyethylene, polypropylene, polyisobutylene, polyisoprene, polyacid anhydride and polycarbonate monomeric subunits, wherein each $R_1$ subunit includes a functional group through which side chains may be attached.

$R_2$ is a straight-chained or branched alkyl, alkoxy, alkylthio or alkylamino group containing from one to ten carbon atoms attached to the monomeric subunit at the functional group of the subunit. The value of p is zero or one, provided that when p is zero, $R_2$ is attached to the functional group by the alkyl moiety and $R_2$ is an alkoxy, alkylthio or alkylamino group. R, D and A are the same as described above with respect to the above-listed five aspects of the present invention, and the ratio of a to b is between about 1:99 and about 50:50.

The NLO compounds of the present invention, including the polymeric materials of the present invention, possess desirable second order NLO properties, and, compared to the prior art, have significantly increased thermal and photochemical stability, attributable to the elimination of aromatic rings linked by pi-bridges. The increase in thermal and photochemical stability is so dramatic that it is now possible to prepare NLO materials that are thermally stable up to 300°C that also possess good second order nonlinearity, photochemical stability, ease of functionalization and improved solubility. Without being bound by any particular theory, it is believed that the five-membered heteroaromatic rings maintain the second order nonlinearity that had been provided by the pi-bridges.

The five-membered heteroaromatic rings are believed to increase the extent of electron delocalization that facilitate the enhancement of the NLO activity as the number of aromatic rings increases. In the absence of five-membered heteroaromatic rings, NLO activity ceases to significantly increase when the total number of aromatic rings is two.

The second order NLO properties of the NLO compounds of the present invention are also not simply the result of the number of aromatic rings. It is believed that the efficient second order NLO properties of the heteroaromatic NLO compounds of the present invention are attributable to the higher degree of pi-delocalization possessed by a five-membered heteroaromatic ring.

The present invention further includes the discovery that the second order NLO properties of structures containing five-membered heteroaromatic ring compounds are significantly increased by the use of particularly strong electron donating groups such as $-N(CH_3)_2$, pyrrolidine, dithiolium, dithiane, piperidine, piperazine and morpholine and particular strong electron accepting groups such as $-NO_2$, dicyanoethylene, tricyanoethylene, cyanonitroethylene, nitroesterethylene, N,N-dialkylbarbituric acids, N,N-dialkylthiobarbituric acids, dinitroethylene and 3-dicyanovinyl-indane-1-sulfone, 1,3-bissulfonyl-indane, indane-1,3-dione, 3-dicyanoindane-1-one, 1,3-bisdicyanovinylindane and

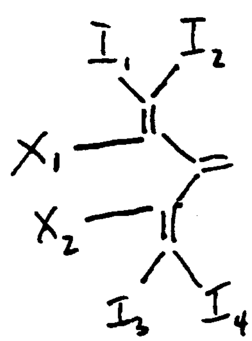

wherein $I_1$, $I_2$, $I_3$ and $I_4$ are independently selected from cyano, nitro, ester, sulfonyl and phosphonyl groups, and $X_1$ and $X_2$ form a saturated or unsaturated five- to eight-membered cyclic ring or two-ring system having five- to eight-membered rings. The improvement in second order NLO properties resulting from the use of strong electron donating and accepting groups with heteroaromatic compounds has been discovered to be significantly greater than the improvement resulting from the use of strong electron donating and accepting groups with compounds having only benzenoid-aromatic rings.

The present invention additionally includes the discovery that when the aromatic ring to which the electron accepting group is to be attached is a five-membered heteroaromatic ring, the strong electron accepting group tricyanoethylene can be readily attached to the heteroaromatic ring by reacting the ring with tetracyanoethylene in a basic solvent at a given temperature. Tetracyanoethylene will not react with corresponding benzenoid structures under these conditions. The tricyanoethylene group is so readily attached that polymeric materials can first be synthesized from monomers having covalently bonded pendant side chains of the NLO materials of the present invention having no electron accepting groups. This alleviates the solubility and reactivity problems associated with particularly strong accepting groups in polymerization reactions, resulting in polymers having high molecular weights and polymer yields as high as 100 percent.

The base polymer is then easily reacted with tetracyanoethylene in a basic solvent at a given temperature to attach the tricyanoethylene group to the pendant side chain. Full attachment of the group is possible with copolymers having up to 40 percent monomeric subunits with pendant side chains of NLO materials.

Therefore, another aspect of the present invention provides a method of preparing a polymer having second order nonlinear optical properties. The method includes the steps of forming a reaction mixture in a basic solvent of tetracyanoethylene and a base polymer having pendant side chains, which polymer has recurring structural units presented by Formula V:

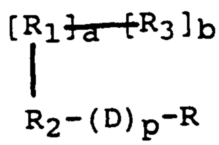

(V)

reacting the mixture at a temperature between about 0 °C and about 150 °C so that R is tricyanovinylated; and recovering the resulting polymer having tricyanovinylated side chains. R, $R_1$, $R_2$, $R_3$, D and p and the ratio of a to b are the same as described above with respect to Formula IV.

Another aspect of the present invention provides a base polymer having pendant side chains capable of being tricyanovinylated to form a polymer having second order nonlinear properties. The base polymer includes recurring structural units represented by Formula V in which R, $R_1$, $R_2$, $R_3$, D and p and the ratio of a to b are the same as described above with respect to Formula V.

The base polymer of Formula V has a high molecular weight, good overall solubility and a low $T_g$. However, the $T_g$ increases upon attachment of the tricyanoethylene group, although the solubility does not significantly suffer. It is possible to take advantage of the low $T_g$ of the base polymer by first forming a thin film of the base polymer and then reacting the film with tetracyanoethylene to attach tricyanoethylene groups to the polymer and produce a film having nonlinear optical properties. This is particularly desirable if the difference in $T_g$ between the base polymer and polymer having nonlinear optical properties result in the base polymer having significantly superior mechanical properties over the polymer having nonlinear optical properties.

In addition to possessing good second order NLO susceptibilities, good solubility and thermal and photochemical stability, the compounds of the present invention have high laser damage thresholds, are easily synthesized and have well-known and understood chemical properties.

## DETAILED DESCRIPTION OF THE PREFERRED EMBODIMENTS

The heteroaromatic NLO compounds of the present invention, once suitably oriented, exhibit a high second order NLO susceptibility. Compounds suitable for use as second order NLO chromophore materials according to the present invention include fused ring systems containing two or three aromatic or heteroaromatic rings, at least one of which is a five-membered heteroaromatic ring, and non-fused ring systems containing from one to four five-membered heteroaromatic rings. Within the present specification "aromatic" rings are defined as aromatic carbocyclic rings such as phenyl groups. "Heteroaromatic" rings

are defined as being limited to aromatic heterocyclic rings, thereby excluding carbocyclic rings such as phenyl groups.

The two- or three-ring fused ring systems can consist entirely of five-membered heteroaromatic rings. Within such fused ring systems, the five-membered heteroaromatic rings possess the structure of Formula II, in which Y is C or N and X is selected from O, N, S and Se. A fused ring system consisting of three five-membered heteroaromatic rings is preferred over a system consisting of two five-membered heteroaromatic rings.

The configuration of the heteroaromatic rings within the fused ring system is not critical, and may be an all "up" configuration as shown in Formula VIa:

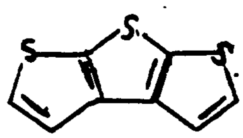

(VIa)

Or the fused ring system may posses an alternating "up" and "down" configuration as shown in Figure VIb:

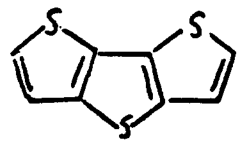

(VIb)

The two or three five-membered heteroaromatic rings may have the same or different heteroatoms.

The fused ring systems of the present invention are not limited to structures containing only five-membered heteroaromatic rings. Two-ring fused ring systems suitable for use with the present invention may also contain a benzene or pyridine ring. Thus, two-ring fused ring systems of the present invention include the following structure:

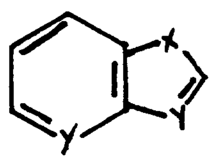

Like the fused ring systems containing all five-membered heteroaromatic rings, Y is independently N or C and X is selected from O, N, S and Se. However, when the two-ring fused ring system contains a benzene ring, Y is limited to carbon.

Three-ring fused ring systems suitable for use with the present invention may contain up to two benzene or pyridine rings, or a benzene and a pyridine ring, in addition to the five-membered heteroaromatic ring. Thus, the three-ring fused ring systems of the present invention include the following structures:

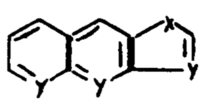
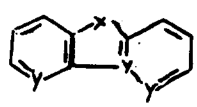
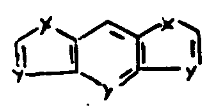
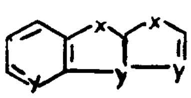
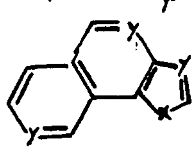
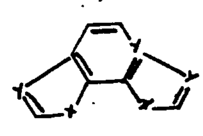

As with the other fused ring systems, Y is independently N or C and X is selected from O, N, S and Se. However, when the three-ring fused ring system has a structure corresponding to Formula III, X is selected

from O, S and Se. For three-ring fused ring systems containing two five-membered heteroaromatic rings, the rings may have the same or different heteroatoms.

When a two- or three-ring system includes pyridine, the pyridine should not be quaternized. Such ionic species cause severe current leakage during the dipole-alignment electric field poling processes.

The fused ring compounds of the present invention are limited to two- and three-ring fused ring systems so as not to hinder the solubility of the NLO compounds in polymer matrices or spin-casting solvents. Three-ring fused ring systems are preferred because of their greater second order nonlinearity.

To induce charge asymmetry, the fused ring system is substituted with an electron donating group, D, and an electron accepting or withdrawing group, A, as in Formula I. The electron donating group and electron accepting group are substituted to ring members of different rings so as to form a delocalized resonance configuration. Positions for substituting electron donating and electron accepting groups to form a delocalized resonance configuration can be readily determined by those of ordinary skill in the art.

The electron donating and electron accepting groups are preferably substituted on five-membered heterocyclic ring members of fused ring ring systems, although this is not essential. For heteroaromatic rings, the electron donating group or electron accepting group is preferably substituted alpha to a heteroatom. The following are examples of typical delocalized resonance configurations. For purposes of illustration, Y is C, but can be readily replaced by N.

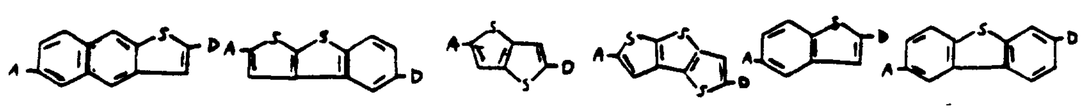

The depicted delocalized resonance configurations are provided to illustrate preferred embodiments of the invention, and not to limit the scope of the claimed invention. The identity of the delocalized resonance configurations, particularly when Y is N, is well understood by those of ordinary skill in the art.

The electron donating and accepting groups that are used to induce charge asymmetry to the fused ring systems of the present invention are essentially conventional to the art of NLO active organic materials. Any functional group capable of releasing electrons into the fused ring systems of the present invention is suitable for use a an electron donating group.

Examples of suitable electron donating groups known in the art include $-NR_5R_6$, $-OR_8$, $-SR_8$, $-TeR_8$, $-SeR_8$, $-CH=NR_4$, $-CH=N-NH_2$, $-CH=N-N(R_5R_6)$ and $-CH=C[N(R_5R_6)]_2$, wherein $R_4$ is hydrogen or an alkyl group containing up to ten carbon atoms, $R_8$ is an alkyl group containing up to six carbon atoms and $R_5$ and $R_6$ are independently selected from hydrogen, alkyl groups containing up to twelve carbon atoms and alkyl groups containing up to twelve carbon atoms having reactive functional groups selected from hydroxyl, ethylene, acetylene, amine, thiol, sulfonic acid, carboxylic acid, or $R_5$ and $R_6$ together form a cyclic group containing up to eight carbon atoms, including groups such as pyrrolidine, piperidine, piperazine and morpholine. Preferably, R is a methyl group, $R_4$ is either hydrogen or a methyl group, and $R_5$ and $R_6$ are independently selected from methyl, ethyl, hexyl, cyclopentyl, cyclohexyl, pyrrolidine, piperidine, piperazine and morpholine.

Another example of suitable electron donating groups are the functional groups:

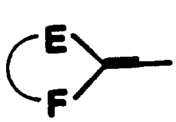 and 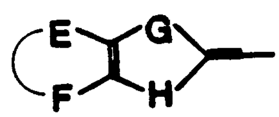

wherein E, F, G and H are members of a saturated or unsaturated five to eight-membered cyclic ring or two-ring system having five to eight-membered rings that are electron donating in nature. E, F, G and H are heteroatoms independently selected from O, N, S, Se and Te. Other suitable electron donating groups include pyrrolidine, piperidine, piperazine, morpholine, dithiane, dithiolium groups such as 1,3-dithiolium, 2-benzo-1,3-dithiolium and 2-ethylenedithio-1,3-dithiolium and the like. Whether or not a ring is electron donating in nature to meet the definition of membership within the group is understood by those of ordinary skill in the art.

Any functional group capable of withdrawing electrons from a fused ring system is suitable for use as an electron accepting group. Examples of suitable electron accepting groups known in the art include $-NO_2$, -CN, -CHO, $-COR_{10}$, $-COOR_{10}$, $PO(OR_{10})_2$, $-SO_2R_{10}$, $-SO_3R_{10}$, $-PO(R_{10})_2$ and

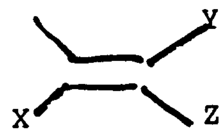

wherein X, Y and Z are independently selected from hydrogen, -CN, $-NO_2$, $-COR_{10}$, $-COOR_{10}$, $-SO_2R_{10}$, $-PO(R_{10})_2$ and $-PO(OR_{10})_2$. $R_{10}$ is an alkyl group containing up to 15 carbon atoms, and preferably is a methyl group. Other suitable electron accepting groups include N,N-dialkylbarbituric acids, N,N-dialkylthiobarbituric acids, 3-dicyanovinylindane-1-sulfone, 1,3-bissulfonylindane, indane-1,3-dione, 3-dicyanovinylindane-1-one, 1,3-bisdicyanovinylindane and

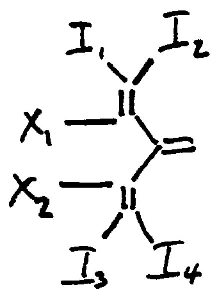

wherein $I_1$, $I_2$, $I_3$ and $I_4$ are independently selected from cyano, nitro, ester, sulfonyl and phosphonyl groups, and $X_1$ and $X_2$ form a saturated or unsaturated five- to eight-membered cyclic ring or two-ring system having five- to eight-membered rings.

Strong electron donating and electron accepting groups are preferred, which significantly increase the second order NLO properties of the compounds of the invention. Examples of strong electron donating groups are $-N(CH_3)_2$, pyrrolidine, dithiane, piperidine, piperazine, morpholine and dithioliums such as 1,3-dithiolium, 2-benzo-1,3-dithiolium and 2-ethylenedithio-1,3-dithiolium. The most preferred strong electron donating group is 2-ethylenedithio-1,3-dithiolium. Examples of strong electron accepting groups are $-C(CN)=C(CN)_2$, $-NO_2$, dicyanoethylene, dinitroethylene, cyanonitroethylene, nitroesterethylene, N,N-dialkylbarbituric acids, N,N-dialkylthiobarbituric acids, 3-dicyanovinylindane-1-sulfone, 1,3-bissulfonyl indane, indane-1,3-dione, 3-dicyanovinylindane-1-one, 1,3-bisdicyanovinylindane and

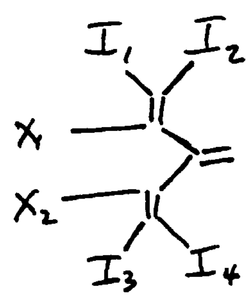

wherein $I_1$, $I_2$, $I_3$ and $I_4$ are independently selected from cyano, nitro, ester, sulfonyl and phosphonyl groups, and $X_1$ and $X_2$ form a saturated or unsaturated five- to eight-membered cyclic ring or two-ring system having five- to eight-membered rings. The most preferred strong electron accepting group is $-C(CN)=C(CN)_2$, a tricyanoethylene or tricyanovinyl group.

A preferred embodiment of the present invention includes a second electron donating group, or a second electron accepting group, or both, attached to the same ring members of the fused ring systems as the respective first electron donating group and the first electron accepting group, so that all of the electron donating and electron accepting groups present and R form a delocalized resonance configuration. The second electron donating or accepting group may be the same or different than the corresponding first electron donating or accepting group. The inclusion of a second electron donating or electron accepting group increases the second order NLO properties of the resulting material as compared to materials having single-substitution of electron donating and electron accepting groups.

The fused ring systems of the NLO compounds of the present invention may optionally be further substituted. Any number of functional groups can be substituted on the rings of the fused ring systems, provided that the groups are not so large or so numerous to cause undesirable steric hindrance effects, the occurrence of which will be clear to those of ordinary skill in the art.

For example, the fused ring systems of the present invention can be functionalized with appropriate substituents to increase the solubility of the materials both in polymer matrices and in spin-casting solvents. Ordinarily, the solubility would decrease as the number of rings in the fused-heteroaromatic compounds increases. Substituents that will increase the solubility of the fused ring systems are well-known to those of ordinary skill in the art. Hydrocarbon side chains such as alkyl group up to 10 carbon atoms in length are preferred substituents.

Compounds suitable for use as second order NLO chromophore materials according to the present invention also include compounds having from one to four non-fused five-membered heteroaromatic rings functionalized with strong electron donating groups, strong electron accepting groups, or both. NLO compositions of the present invention therefore can also have a structure corresponding to Formula I wherein R is selected from heteroaromatic ring compounds represented by the structure of Formula IIa in which Y is N or C, X is selected from O, N, S and Se and q is an integer from one to four, inclusive.

D is an electron donating group, A is an electron accepting group, and D and A and the one to four rings of R form a delocalized resonance configuration. Either D is a strong electron donating group or A is a strong electron accepting group or both D and A are strong electron donating and electron accepting groups, respectively. Suitable strong electron donating groups and strong electron accepting groups are the same as those described above with respect to the fused ring NLO compounds of the present invention.

The non-fused five-membered heteroaromatic ring NLO compounds of the present invention may also optionally be further substituted so as, for example, to improve solubility. When more than one non-fused heteroaromatic ring is present, D and A are attached to different rings and second electron donating groups and second electron accepting groups may optionally be employed. Therefore, NLO compounds in accordance with the present invention may have the following structures:

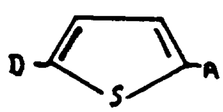

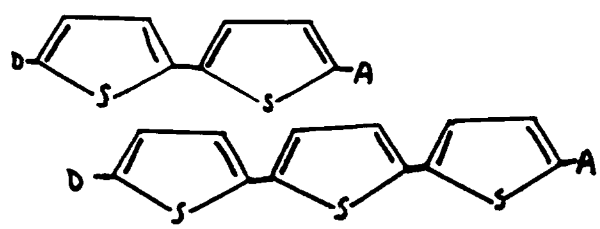

The ring structures of two or three aromatic rings, at least one of which is a five-membered heteroaromatic ring, upon which the NLO compounds of the present invention are based, are prepared by well-known methods widely reported in the journal art. For example, Goldfarb et al., Bull. Acad. Sci. U.S.S.R., 46 (1965) discloses the preparation of thieno[3,2-b] thiophene. Goldfarb et al., Bull. Acad. Sci. U.S.S.R., 310 (1963) discloses the preparation of thieno[2,3-b] thiophene.

DeJong et al., J. Org. Chem., 36, 1645 (1971) disclose the preparation of dithieno[3,2-b:2',3'-d] thiophene, dithieno[2,3-b:3',2'-d]thiophene, dithieno [3,4-b:3',4'-d]thiophene and dithieno[2,3-b:3',4'-d]-thiophene. DeJong et al., J. Org. Chem., 36, 1998 (1971) disclose the preparation of dithieno[3,2-b:3',4'-d]-thiophene and dithieno[2,3-b:2',3'-d]thiophene.

Farnier et al., Can. A. Chem., 56, 1429 (1978) discuss the preparation of thieno[3,2-b]pyrrole, furaro[3,2-b]pyrrole, seleno[3,2-b]pyrrole, thieno[2,3-b]pyrrole, seleno[2,3-b]pyrrole and pyrrolo[2,3-b]pyrrole. Cagniant et al., Adv. Heterocycl. Chem., 18, 337 (1975) discloses a preparation of benzo[b]furan. Scronston, Adv. Heterocycl. Chem., 29, 171 (1981) discloses the preparation of benzo[b]thiophene. Berlin et al., Synth. Met., 41, 2393 (1991) discloses the preparation of compounds having the formula:

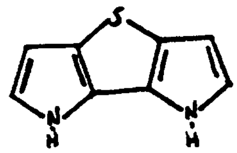 and 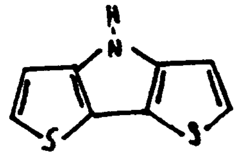

Some of the ring structures are commercially available.

A variety of electron acceptor groups such as tricyanoethylene, dicyanoethylene, cyanoesterethylene, and the like, can be substituted to the fused and non-fused ring systems containing one or more five-membered heteroaromatic rings using conventional methods. While the methods may be conventional, it has unexpectedly been discovered that the tricyanoethylene acceptor group can be directly attached to the five-membered heteroaromatic rings of the structures of the present invention, a reaction that does not readily occur with benzene rings. Likewise, a variety of electron donating groups can be substituted using conventional methods.

The NLO compounds of the present invention can be formed into a nonlinear optical material by combining the NLO compounds with a chemically inert medium. For example, the NLO compounds can be layered on a substrate such as glass, silica or polymeric materials, as described in U.S. Patent No. 4,894,186 to Gordon, the disclosure of which is hereby incorporated herein by reference thereto. In another embodiment, a nonlinear optical medium can be formed by blending the NLO compounds of the present invention with a host thermoplastic polymer. Suitable host thermoplastic polymers include polyacrylates, polymethacrylates, polyacrylamides, polyimides, polycarbonates, polyesters, polyamides, polystyrenes and the like. This combination is also described in U.S. Patent No. 4,894,186, the disclosure of which is also hereby incorporated herein by reference thereto.

The NLO compounds of the present invention can also be covalently attached as side chains to the monomeric subunits of polymers. Polymers should be chosen for use with the present invention having monomeric subunits that have reactive functional groups for attachment of the side chains.

The polymer should also have excellent optical transparency, good film-forming characteristics, a low dielectric constant and a relatively high $T_g$ for stable dipole orientation of the side chains. Other properties will come into consideration, depending upon the particular end-use requirements of the material; however, these properties are well understood by those of ordinary skill in the art.

One class of polymers suitable for use with the present invention are polymers and copolymers, the monomeric subunits of which are derived from vinyl monomers such as acrylates, acrylamides, vinyl halides, acrylonitriles, ethylene, propylene, isobutylene, isoprene, acid anhydrides, styrenes, vinyl alcohols and vinyl acetates. Examples of other polymers suitable for use with the present invention include polyimides, polyamides, polycarbonates, polyesters, polyphenylene ethers, polyetherimides, polyether ketones and polyether etherketones.

The polyacrylates suitable for use with the present invention include alkyl branched polyacrylates such as polymethyl methacrylate. Likewise, the polyacrylamides suitable for use with the present invention include alkyl branched polyacrylamides such as polymethacrylamide, and the polyacrylonitriles include alkyl branched polyacrylonitriles such as polymethacrylonitrile.

Those of ordinary skill in the art are capable of identifying the functional groups of the polyacrylates, polyimides, polyamides, polyacrylamides, polyvinyl halides, polyacrylonitriles, polyvinyl alcohols, polyvinyl acetates, polyesters, polyphenylene ethers, polyetherimides, polyether ketones, polyether etherketones, poly(acid anhydrides) and polycarbonates to which the NLO compounds of the present invention can be attached to form side chains by conventional addition and condensation reactions. Although the monomeric side units of polystyrene, polyethylene, polypropylene, polyisobutylene and polyisoprene do not have such functional groups, such monomeric subunits can first be functionalized to provide a reactive group for the attachment of the NLO compound, such as the chloromethylation of polystyrene and the subsequent conversion to the more reactive iodomethyl derivative set forth in U.S. Patent No. 4,935,292 to Marks, the disclosure of which is herein incorporated by reference thereto.

Alternatively, a functionalized derivative of these polymers can be used as a starting material, such as the poly(p-hydroxystyrene), the use of which is also disclosed by U.S. Patent No. 4,935,292.

Attachment of the NLO compounds to the monomeric subunits results in a polymer having side chains corresponding to Formula IV, wherein $R_1$ and $R_3$ represent the monomeric subunits of the above-described polymers, which may be the same or different, and D and A are the same as described above, and are

attached to R as described above, with respect to Formula I. $R_2$ is a straight-chained or branched alkyl, alkoxy, thioalkyl or aminoalkyl group containing from one to ten carbon atoms and p is zero or one. When p is zero, $R_2$ replaces D on the ring structure and is attached to the functional group by the alkyl moiety. In such circumstances, $R_2$ is an alkoxy, alkylthio or alkylamino group. The ratio of a to b represents the degree to which the monomeric subunits of the polymer are substituted by NLO side chains. In the present invention, the ratio of a to b is between about 1:99 and about 50:50. Preferably, the ratio of a to b is between about 5:95 and 40:60 and most preferably, it is about 25:75. The ratio of a to b should not substantially exceed 30:70 in order that the polymer remains soluble in solvents utilized in the preparation of NLO materials.

R is the same as described above with respect to the above-listed five aspects of the present invention. In particular, the letter R can be a fused ring system consisting of two or three five-membered heteroaromatic rings, in which each such heteroaromatic ring independently has a structure corresponding to Formula II, wherein X and Y are the same as described above with respect to Formula II.

R can also be a fused ring system consisting of one five-membered heteroaromatic ring and one benzene or pyridine ring, wherein the five-membered heteroaromatic ring has a structure represented by Formula II in which X and Y are the same as described above with respect to Formula II, with the proviso that when the ring system includes a benzene ring, Y is C. R can also be a fused ring system consisting of one five-membered heteroaromatic ring and two rings independently selected from benzene and pyridine rings, wherein the five-membered heteroaromatic ring has a structure represented by Formula II in which X and Y are the same as described above with respect to Formula II, with the proviso that X is selected from O, S and Se when R has a structure represented by Formula III.

R can also be a fused ring system consisting of two five-membered heteroaromatic rings and a benzene or pyridine ring, wherein each five-membered heteroaromatic ring has a structure represented by Formula II in which X and Y are the same as described above with respect to Formula II. R can also be from one to four non-fused five-membered heteroaromatic ring functionalized with a strong electron donating group, a strong electron accepting group, or both. The non-fused five-membered heteroaromatic ring structure is represented by Formula Ia in which X, Y and q are the same as described above with respect to Formula II. D or $R_2$ and A are attached to R as described above with respect to Formula II for D and A. Suitable strong electron donating and electron accepting groups are the same as described above with respect to the fused ring systems of the present invention.

$R_2$ functions to attach the NLO compounds to the functional groups of the monomeric subunits of the polymers to form NLO side chains. While $R_2$ is a straight-chained or branched alkyl, alkoxy, thioalkyl or aminoalkyl group containing from one to ten carbon atoms, preferably, $R_2$ will contain from four to six carbon atoms. Stated another way, $R_2$ is selected from $(-CH_2-)_x$, $(CH_2-R_9-)_x$, $(-CH_2-CH_2-R_9-)_{x/2}$, $(-CH_2-CH_2-CH_2-R_9-)_{x/3}$, wherein $R_9$ is selected from O, S and NH and x is from one to ten, preferably from four to six, with the proviso that for $(-CH_2-CH_2-R_9-)$, x is a multiple of two, and for $(CH_2-CH_2-CH_2-R_9-)$, x is a multiple of three. $R_2$ additionally functions to increase the solubility of the polymer in the chemically inert medium or solvents utilized.

In preferred NLO polymers, $R_1$ and $R_3$ are independently selected from monomers of ethylene, acrylates, alkyl-branched acrylates, acrylamides, alkyl-branched acrylamides, styrenes, $\alpha$-alkyl styrenes, vinyl acetate, ether ketones and ether etherketones. When $R_1$ or $R_3$ is a styrene monomer, the aromatic ring may be further substituted by one or more hydroxyl or alkyl groups, provided that the groups are not so large or so numerous to cause undesirable steric hindrance effects, the occurrence of which will be clear to those of ordinary skill in the art.

In the most preferred NLO polymers, $R_1$ and $R_3$ are independently selected from monomers of acrylates, methacrylates, ether ketones and ether etherketones.

The base polymers of the present invention have recurring structural units corresponding to the recurring structural units of the NLO polymers of the present invention, but without the electron accepting groups. Therefore, base polymers in accordance with the present invention have recurring structural units represented by Formula V, in which R, $R_1$, $R_2$, $R_3$, D and p are the same as described above with respect to Formula IV. $R_1$ and $R_3$ may be the same or different, as described above with respect to the NLO polymers of Formula IV, with the same a to b monomer ratio as described above with respect to Formula IV. As with the polymers of Formula IV, in the preferred base polymers, $R_1$ and $R_3$ are independently selected from monomers of ethylene, acrylates, alkyl-branched acrylates, acrylamides, alkyl-branched acrylamides, styrenes, $\alpha$-alkyl styrenes, vinyl acetates, ether ketones and ether etherketones. $R_1$ and $R_3$ for the most preferred base polymers are independently selected from monomers of acrylates, methacrylates, ether ketones and ether etherketones.

The polymerization of the base polymers and polymeric NLO materials of the present invention is essentially conventional and is readily understood by those of ordinary skill in the art. Depending on the material in question, in some cases, it is preferable first to polymerize the polymer and then attach the side chains to the functional groups, in other cases it is preferable to synthesize a monomer having an NLO side chain covalently attached thereto to be copolymerized with a monomer having no side chain.

A preferred method for preparation of the polymeric NLO materials of the present invention, however, first synthesizes a monomer having a pre-NLO side chain covalently attached thereto. The pre-NLO side chain is defined as an NLO side chain having no electron accepting group. Once the monomer having a pre-NLO side chain is copolymerized with a monomer having no NLO or pre-NLO side chain, the resulting base polymer can be reacted to attach electron accepting groups to the pre-NLO side chains to provide a polymeric NLO material. The synthesis of monomers having pre-NLO side chains and the copolymerization of same with a monomer having no NLO or pre-NLO side chains is essentially conventional and well understood by those of ordinary skill in the art. The base copolymer is then reacted with an electron accepting group. The preferred electron accepting group is tetracyanoethylene, which tricyanovinylates the pre-NLO side chains. The base polymer can be reacted with tetracyanoethylene in a basic solvent at an elevated temperature to achieve such tricyanovinylation.

For polymers having monomeric subunits such as acid anhydrides, epoxides, acid halides, isocyanates, carboxylic acids, esters, sulfonic acids and amines, the pre-NLO side chain can be directly attached to the polymer, rather than first synthesizing a monomer having a pre-NLO side chain that is then polymerized with an unsubstituted monomer to form a pre-NLO base polymer. For example, pre-NLO side chains can be reacted with poly(styrene maleic anhydride) to form a poly(styrene maleimide) having pre-NLO side chains. The method by which pre-NLO side chains can be directly attached to polymers is also essentially conventional and well understood by those of ordinary skill in the art. The polymer having pre-NLO side chains can then be reacted to attach electron accepting groups such as tricyanovinyl groups to the pre-NLO side chains to provide a polymeric NLO material.

For the tricyanovinylation of pre-NLO base polymers, suitable basic solvents for the reaction of tetracyanoethylene and the base polymer include N,N-dimethylformamide (DMF), pyridine, N,N-dimethylacetamide, N-methyl pyrrolidone, tertiary amines and the like. The preferred solvent is DMF. A reaction mixture is prepared by dissolving the polymer and the tetracyanoethylene in one or more of the above solvents. The reaction mixture is heated to a temperature between about 50 °C and about 140 °C, preferably to about 100 °C, to obtain the tricyanovinylated polymers.

The degree of tricyanovinylation of the polymer is limited only by the number of pendant pre-NLO groups available for tricyanovinylation. Therefore, a slight equivalent excess of the tetracyanoethylene over the polymer should be used.

As noted above, the reaction of the polymer and the tetracyanoethylene can be carried out at temperatures in the range of from about 50 °C to about 140 °C. Higher temperatures will result in an increased rate of reaction, and even higher rates can be achieved by pressurizing the reaction vessel to elevate the boiling point of the solvent, allowing the reaction to proceed at an even higher temperature. However, a reaction temperature of 100 °C is preferred to minimize inter- and intra-molecular cross reactions.

To insure uniform mixing of the polymer and the tetracyanoethylene, the reaction mixture should be maintained at a constant state of mild agitation. It is also preferred that the reaction mixture be maintained under an atmosphere of an inert gas.

Once the reaction is complete, the NLO polymer is precipitated with a lower alkyl alcohol, such as methanol or isopropanol, filtered and dried under vacuum. The polymer can then be further purified by conventional methods, typically by repeated dissolution and reprecipitation from the lower alkyl alcohol.

A general procedure employed to synthesize acrylate monomers with fused ring pre-NLO side chains, followed by copolymerization with an unsubstituted acrylate monomer, and activation of the pre-NLO side chains, is illustrated below:

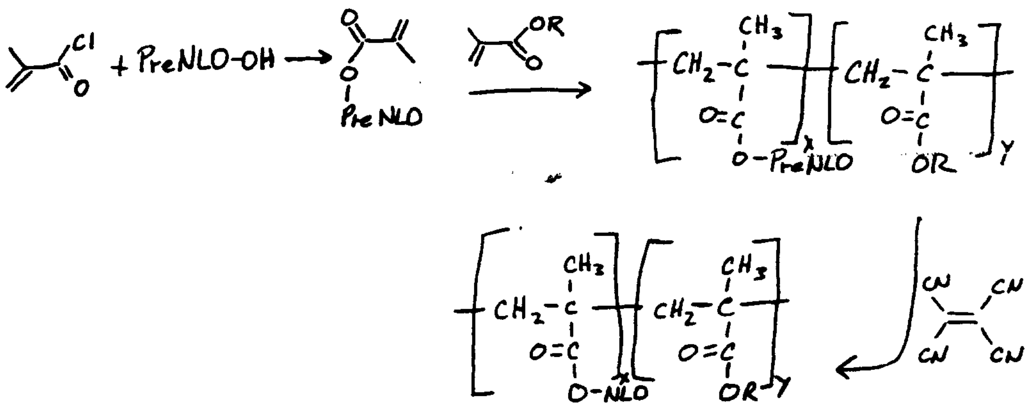

The foregoing reactions can also be employed with non-fused ring systems of one to four five-membered heteroaromatic rings.

The polymeric materials having NLO active side chains are recovered and purified by conventional means known to those of ordinary skill in the art. Films of the polymers may be formed by spin coating, after which the films may be repetitively annealed prior to poling at an elevated temperature near the $T_g$ of the material. Following annealing, the dipoles of the side chains may be aligned by application of an intense electric field (0.2 - 1.0 MV $cm^{-1}$) at temperatures near the $T_g$. The foregoing sequence of spin coating, annealing and poling is essentially conventional and disclosed in U.S. Patent No. 4,935,292, the disclosure of which is hereby incorporated herein by reference thereto.

It is disclosed in U.S. Patent No. 4,935,292 and SPIE Proceding No. 1147, 74-83 (1989) that further stabilization of the NLO side chain alignment can be achieved by a radiation-induced or chemical-induced cross-linking of the polymer matrix. This process is also essentially conventional, the disclosure of which in U.S. Patent No. 4,935,292 is also hereby incorporated herein by reference thereto.

The preferred base polymers and NLO-active polymeric materials of the present invention typically have weight-average molecular weights between about 5,000 and about 300,000 daltons measured by GPC or light scattering. The incorporation of the tricyanovinyl group increases the $T_g$'s of the precursor polymers.

The electro-optic coefficient of an NLO-active poled polymer film is proportional to the product of the molecular second order nonlinear optical susceptibility coefficient, $\beta$, and the molecular ground state electric dipole moment, $\mu$. The molecular $\beta$ is dependent upon the frequency at which the measurement is performed due to the resonance effect near the absorption peak. A method to compare molecules with different absorption properties by extrapolation of the $\beta$ value measured at a specific frequency to zero frequency using a two-level model is disclosed by Singer, J. Opt. Soc. Am., B6, 1339-50 (1989). The $\beta$ value at the extrapolated zero frequency is defined $\beta_0$. The NLO-active molecules of the present invention can exhibit values of the $\beta\mu$ product as high as about 4,000 in units of $10^{-48}$esu measured at a wavelength of 1907 nm.

Thus, it can be appreciated that the present invention provides NLO compounds combining second order nonlinear optical properties with the physical, mechanical and optical properties required of an optical material, together with thermal and photochemical stability. The following examples are further illustrative of the present invention, and are not to be construed as limiting the scope thereof. Unless otherwise indicated, materials were obtained from Aldrich Chemical Supply. All parts and percentages are by weight unless expressly indicated to be otherwise.

EXAMPLES

EXAMPLE 1

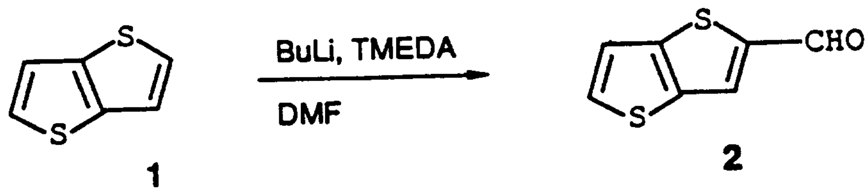


1 2

2-formyl thienylthiophene **2** was prepared by adding n-BuLi (2.5 M, 8.46 mL, 21.4 mmol) to a stirred solution of thienylthiophene (**1**, 3.0 g, 21.4 mmol) in 60 mL tetrahydrofuran (THF) at 0 ° C. The resulting mixture was maintained at 0 ° C for thirty minutes. To this, tetramethyl ethylenediamine (3.2 mL, 21.4 mmol) was added and the solution was warmed to room temperature for one hour. The mixture was then recooled to 0 ° C and N,N'-dimethylformamide (DMF, 5.0 mL, excess) was added and the mixture was again allowed to warm to room temperature. After approximately one hour the reaction was quenched with 10 mL of water and stirred overnight. The THF was removed in vacuo and 100 mL of water was added. The product was extracted out of the aqueous layer using methylene chloride (2 x 200 mL). The combined extracts were dried ($Na_2SO_4$), concentrated and chromatographed (hexane:dichloromethane, 1:1) to yield the aldehyde **2** -(2.29 g, 64.0% yield) as a white solid.

EXAMPLE 2

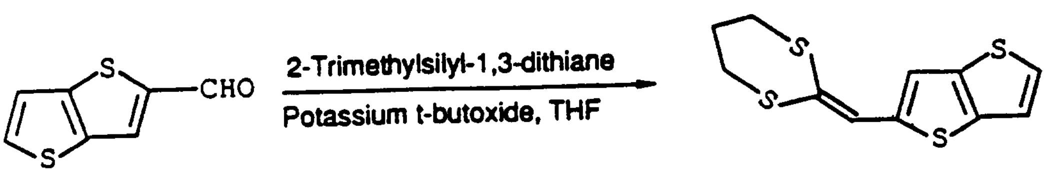


2

The 1,3-dithiane-substituted thienylthiophene **3** was prepared by adding the aldehyde **2** of Example 1 (1.0 g, 6 mmol) to a solution of 2-trimethylsilyl-1,3-dithiane (1.15 g, 6 mmol) and potassium-t butoxide (0.71 g, 6.3 mmol) in 50 mL THF at 0 ° C. After the addition, the mixture was stirred at room temperature under an argon atmosphere for two hours. The reaction mixture was quenched with 100 mL water, and the THF was removed under reduced pressure. The aqueous phase was extracted, dried and concentrated as in Example 1. The solvent concentration was followed by column chromatography (silica gel/hexane), to yield the 1,3-dithiane substituted thenyl thiophene **3** (1.3 g, 80% yield).

EXAMPLE 3

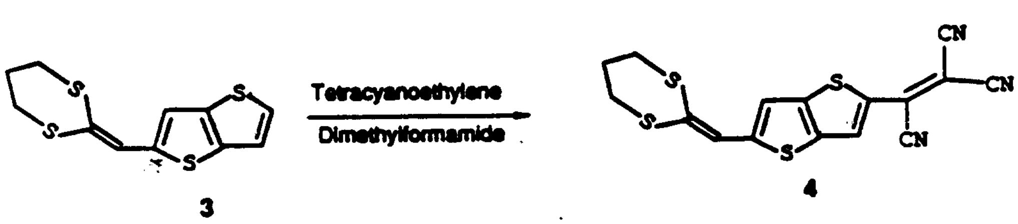


3 4

Tricyanovinylated 1,3-dithiane substituted thienylthiophene **4** was prepared by mixing the 1,3-dithiane substituted thienylthiophene **3** of Example 2 (1.0 g, 3.7 mmol) with tetracyanoethylene (0.5 g, 4 mmol) in 20 mL DMF at 0 ° C. The reaction mixture was stirred at room temperature for three hours and at 50 ° C for another twenty hours. The reaction mixture was quenched with 100 mL water, and the resulting dark blue colored aqueous solution was extracted with methylene chloride (4 x 250 mL). After washing the organic layer several times with water, the solvent was evaporated and dried as in Example 1. Evaporation of the solvent followed by column chromatography (silica gel, dichloromethane:hexane, 1:1) yielded the dark green

tricyanovinylated 1,3-dithiane substituted thienylthiophene **4** (0.41 g, 30% yield).

EXAMPLE 4

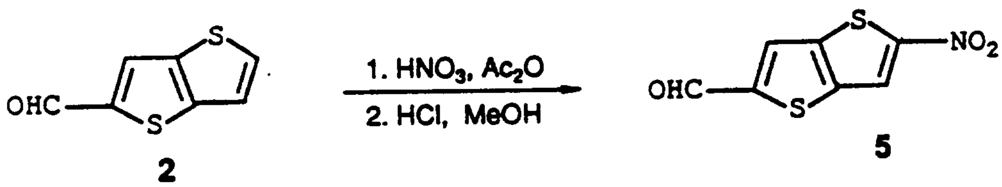


Nitroaldehyde **5** was prepared by first adding nitric acid (0.534 g, 6.0 mmol) dropwise to an ice-cooled solution of acetic anhydride (6 mL). This mixture was stirred for 15 minutes at 0°C and then slowly added to a suspension of the aldehyde **2** (1.0 g, 5.94 mmol) of Example 1 in acetic anhydride (6 mL) at 0°C. After one hour the solution was poured onto 100 g ice with vigorous stirring and left overnight. The red solid that precipitated was extracted with dichloromethane (2 x 100 mL). The dichloromethane solutions were combined, dried ($Na_2SO_4$), concentrated in vacuo and flashed (medium pressure, dichloromethane/hexane:2.5/1.5) to yield a diacetate (1.0 g, 50% yield) as a pale yellow solid. This solid was placed in a 50/50 mixture of water/methanol (100 mL) and acidified with 20 mL hydrochloric acid. The suspension was heated (everything went slowly into solution) for approximately one hour. (The exact time will depend upon the amount of material.) Upon cooling, the nitroaldehyde **5** precipitated and was collected by vacuum filtration to give a crystalline yellow solid (0.54 g, 84% yield).

EXAMPLE 5

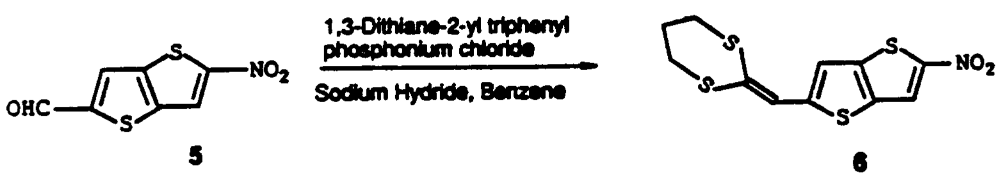


1,3-dithiane substituted nitroaldehyde **6** was prepared by adding the nitroaldehyde **5** of Example 4 (1.5 g, 7 mmol) to a solution of 1,3-dithiane-2-yl triphenylphosphonium chloride (3.0 g, 7.1 mmol) and sodium hydride (0.17 g, 7.1 mmol) in 100 mL benzene at room temperature under an argon atmosphere. The reaction mixture was stirred at room temperature for one hour, and at 60°C for another five hours. Quenching the reaction mixture as in Example 2 resulted in a red colored solid suspension. Extraction of the aqueous solution with dichloromethane as in Example 2, followed by evaporation of the solvent yielded the 1,3-dithiane substituted nitroaldehyde **6** (1.54 g, 70% yield).

EXAMPLE 6

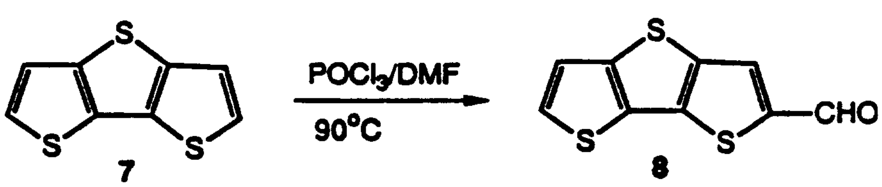


2-formyl dithienylthiophene **8** was prepared by first adding phosphorus oxychloride (3.44 g, 0.022 mmol) dropwise at 0°C to 20 mL DMF. The resulting mixture was stirred at 0°C for two hours. Dithenylthiophene **7** (4.0 g, 0.020 mmol) in 10 mL DMF was added slowly, and the reaction mixture was heated to 90°C for three hours. After cooling, the solution was poured onto 500 g of ice and the resulting material was hydrolyzed with potassium carbonate (16 g, 5 x excess). The basic mixture was extracted with dichloromethane (3 x 200 mL), the organic layers were combined, dried ($Na_2SO_4$), and the solvent was

removed in vacuo. The brown residue was chromatographed (medium pressure, hexane/dichloromethane : 3/1) to yield a white solid 2-formyl dithienylthiophene **8** (3.75 g, 80.1% yield).

EXAMPLE 7

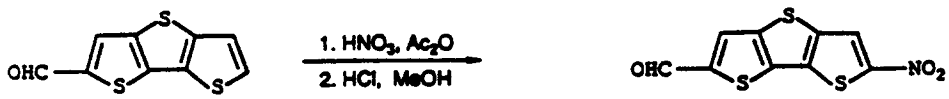


8 9

Nitroaldehyde **9** was prepared by first adding nitric acid (0.245 g, 2.78 mmol) dropwise to an ice-cooled 4 mL solution of acetic anhydride. The mixture was stirred for 15 minutes at 0 ° C and slowly added to a suspension of the 2-formyl dithienylthiophene **8** of Example 6 (0.5 g, 2.3 mmol) in 5 mL acetic anhydride at 0 ° C. After one hour the solution was poured onto 100 g ice with vigorous stirring and left overnight. The red solid that precipitated was extracted with dichloromethane (2 x 50 mL). The dichloromethane solutions were combined, dried ($Na_2SO_4$) concentrated in vacuo and flashed (medium pressure, dichloromethane/hexane:1/1) to yield a diacetate (0.45 g, 60% yield) as a pale yellow solid. This solid was placed in 60 mL of a 50/50 mixture of water/methanol and acidified with 10 mL hydrochloric acid. The suspension was heated for approximately one hour. (The total time will depend upon the amount of material.) Upon cooling, a mustard yellow nitro substituted dithienylthiophene-2-carboxaldehyde **9** was collected by vacuum filtration (0.4 g, 90% yield).

EXAMPLE 8

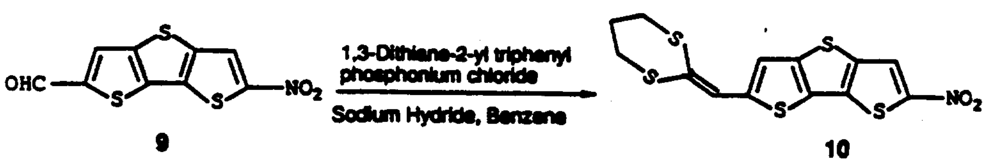


9 10

Nitro substituted dithienyl thiophene-2-(1,3-dithiane) **10**, was prepared by adding the nitro substituted dithienylthiophene-2-carboxaldehyde **9** of Example 7 (1.0 g, 3.7 mmol) to a solution of 1,3-dithiane-2-yl triphenylphosphonium chloride (1.6 g, 3.78 mmol) and sodium hydride (0.09 g, 3.8 mmol) in 50 mL benzene. The reaction mixture was heated at 60 ° C for twenty hours. The reaction mixture was quenched with water as in Example 5, resulting in a red colored solid suspension. Filtration followed by washings with hexane (2 x 50 mL) yielded the nitro substituted dithienyl thiophene-2-(1,3-dithiane) **10** (0.96 g, 70% yield).

EXAMPLES 9-12

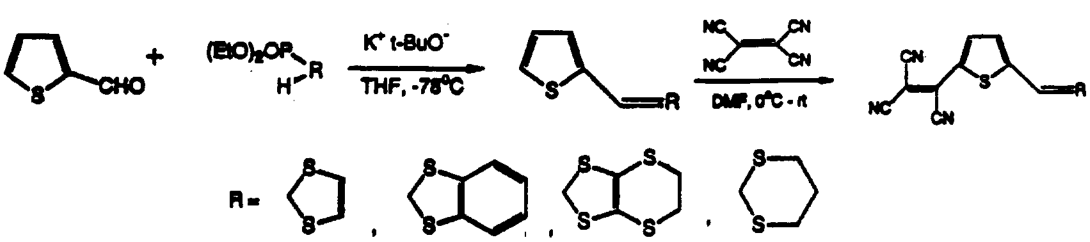


11 12 13 14

EXAMPLE 9

2-(1,3-dithiolium) thiophene **11** was prepared by cooling a mixture of 2-thiophene carboxaldehyde (1.12 g, 10.0 mmol) and 1,3-dithiolium phosphonate ester (2.90 g, 10.0 mmol) to -78 ° C in 25 mL THF under an argon atmosphere. The 1,3-dithiolium phosphonate ester was prepared as described in Tet. Lett., 41, 3695

(1976). Potassium t-butoxide (1.12 g, 10.0 mmol) was added and the mixture was stirred overnight and allowed to warm to room temperature. The THF was removed in vacuo and the resulting oil was redissolved in 100 mL dichloromethane. The dichloromethane was washed with water (2 x 75 mL), dried ($Na_2SO_4$) and concentrated onto 2.5 g silica gel. The mixture was placed onto a medium pressure column and flashed with a gradient of hexane/dichloromethane ranging from 5/1 to 1/1 to yield a 2-(1,3-dithiolium) thiophene (2.3 g, 90% yield).

Tricyanovinylation of 2-(1,3-dithiolium) thiophene was accomplished by gradually adding (approx. 5-10 min.) tetracyanoethylene (0.64 g, 5.0 mmol) to a stirred solution of the 2-(1,3-dithiolium) thiophene in 25 mL DMF at 0 °C under an argon atmosphere. The reaction mixture was slowly warmed to room temperature overnight. The resulting mixture was poured into 200 mL of water and extracted with dichloromethane (2 x 100 mL). The dichloromethane layers were combined, dried ($Na_2SO_4$), and concentrated onto silica gel. The mixture was placed onto a medium pressure column and flashed with a gradient of hexane/dichloromethane ranging from 5/1 to 1/1 to yield the tricyanovinylated 2-(1,3-dithiolium) thiophene (0.69 g, 40% yield).

EXAMPLE 10

2-(benzo-1,3-dithiolium) thiophene **12** was prepared by reacting 2-thiophene carboxaldehyde (1.12 g, 10.0 mmol) and benzo-1,3-dithiolium phosphonate ester (2.40 g, 10.0 mmol) as in Example 9. The benzo-1,3-dithiolium phosphonate ester was prepared as described in J. Org. Chem., 39, 2457 (1974). The procedure yielded 2-(benzo-1,3-dithiolium) thiophene **12** (1.48 g, 75% yield). The 2-(benzo-1,3-dithiolium) thiophene was tricyanovinylated according to the procedure of Example 9 (0.37 g, 25% yield).

EXAMPLE 11

2-(ethylenedithio-1,3-dithiolium)thiophene **13** was prepared by reacting a mixture of 2-thiophene carboxaldehyde (1.12 g, 10.0 mmol) and ethylenedithio-1,3-dithiolium phosphonate ester (3.30 g, 10.0 mmol) according to the procedure of Example 9. The ethylenedithio-1,3-dithiolium phosphonate ester was prepared as described in Synthesis, 26, 24 (1991). The reaction yielded 2-(ethylene-dithio-1,3-dithiolium) thiophene **13** (1.79 g, 70% yield). The 2-(ethylenedithio-1,3-dithiolium) thiophene was tricyanovinylated according to the procedure of Example 9 (89.4 mg, 5% yield).

EXAMPLE 12

2-(1,3-dithiane) thiophene **14** was prepared by reacting 2-thiophene carboxaldehyde (1.12 g, 10 mmol) and 1,3-dithiane-2-yl triphenyl phosphonium iodide (4.46 g, 10 mmol) according to the procedure of Example 9. The 1,3-dithiane-2-yl triphenyl phosphonium chloride was obtained from Lancaster of Windham, New Hampshire and used without purification. The reaction yielded 2-(1,3-dithiane) thiophene (3.10 g, 91% yield). The 2-(1,3-dithiane) thiophene **14** was tricyanovinylated according to the procedure of Example 9 (0.90 g, 50% yield).

EXAMPLES 13-16

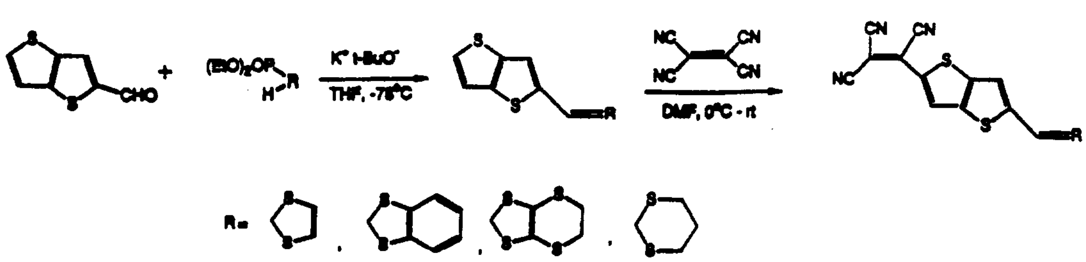


15 16 17 18

EXAMPLE 13

2-(1,3-dithiolium)thienyl[3,2-b]thiophene **15** was prepared by reacting the 2-formyl thienylthiophene **2** of Example 1 (1.68 g, 10.0 mmol) and the 1,3-dithiolium phosphonate ester of Example 9 according to the

procedure of Example 9. The reaction yielded 2-(1,3-dithiolium) thienyl[3,2-b]thiophene **15** (2.16 g, 85% yield). The 2-(1,3-dithiolium) thienyl[3,2-b]thiophene was tricyanovinylated as in Example 9 (0.35 g, 10% yield).

EXAMPLE 14

2-(benzo-1,3-dithiolium) thienyl[3,2-b] thiophene **16** was prepared by reacting the 2-formyl thienylthiophene **2** of Example 1 with the benzo-1,3-dithiolium phosphonate ester of Example 10 (2.40 g, 10 mmol) according to the procedure of Example 9. The reaction yielded 2-(benzo-1,3-dithiolium)thienyl[3,2-b] thiophene **16** (2.58 g, 85% yield). The 2-(benzo-1,3-dithiolium)thienyl[3,2-b]thiophene was tricyano-vinylated according to the procedure of Example 9 (0.61 g, 15% yield).

EXAMPLE 15

2-(ethylenedithio-1,3-dithiolium)thienyl [3,2-b]thiophene **17** was prepared by reacting the 2-formyl thenylthiophene **2** of Example 1 with the ethylenedithio-1,3-dithiolium phosphonate ester of Example 11 (3.30 g, 10.0 mmol) according to the procedure of Example 9. The reaction yielded 2-(ethylenedithio-1,3-dithiolium) thienyl [3,2-b]thiophene **17** (2.58 g, 75% yield). The 2-(ethylenedithio-1,3-dithioleum) thienyl[3,2-b]thiophene was tricyanovinylated according to the procedure of Example 9 (89.1 mg, 2% yield).

EXAMPLES 16-19

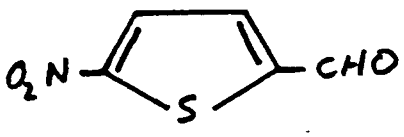

19

In Examples 16-19, the aldehyde **19** (Aldrich Chemical, 1.57 g, 10.0 mmol) was reacted with the 1,3-dithiolium phosphonate ester of Example 9 (2.90 g, 10.0 mmol), or the benzo-1,3-dithiolium phosphonate ester of Example 10 (2.40 g, 10.0 mmol), or the ethylenedithio-1,3-dithiolium phosphonate ester of Example 11 (3.30 g, 10.0 mmol) or the 1,3-dithiane-2-yl triphenyl phosphonium iodide of Example 12 (4.46 g, 10 mmol) according to the procedure of Example 9. Yields of 85%, 76%, 50% and 80%, respectively, were obtained.

EXAMPLES 20-23

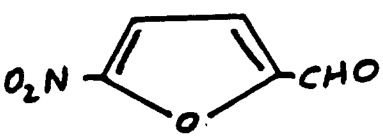

20

In Examples 20-23, aldehyde **20** (Aldrich Chemical, 1.41 g, 10.0 mmol) was substituted for aldehyde **19** of Examples 16-19 and reacted with the phosphonate esters or triphenyl phosphonium iodide of Examples 9-12 as in Examples 16-19. Yields of 60%, 65%, 42% and 80%, respectively, were obtained.

EXAMPLES 24-27

In Examples 24-27, the aldehyde **5** of Example 4 (2.14 g, 10 mmol) was substituted for aldehyde **19** of Examples 16-19 and reacted with the phosphonate esters and triphenyl phosphonium iodide of Examples 9-12 as in Examples 16-19. Yields of 30%, 55%, 25% and 70%, respectively, were obtained.

EXAMPLES 28-31

In Examples 28-31, the aldehyde **9** of Example 7 (2.70 g, 10 mmol) was substituted for aldehyde **19** of Examples 16-19 and reacted with the phosphonate esters and triphenyl phosphonium iodide of Examples 9-12 as in Examples 16-19. Yields of 15%, 48%, 30% and 70%, respectively, were obtained.

Second order NLO properties of structures disclosed in the present invention were determined by standard electrical field induced second harmonic generation measurements. In the measurement, a static electric field is applied to align the NLO molecules which are dissolved in a suitable solvent. The measurement of the second harmonic, which is generated by shining a single frequency laser beam through the electric field aligned solution, determines the value of $\beta\mu$. Representative $\beta\mu$ values for the compounds of Examples 3, 9-12, 20 and 25 are depicted in the table below:

TABLE I

| Example | Electronic Absorption λmax(nm) | NLO Coefficient $\beta\mu$ x $10^{-48}$ esu |
|---|---|---|
| 3 | 600 | 2200 |
| 9 | 625 | 1600 |
| 10 | 604 | 1350 |
| 11 | 638 | 2200 |
| 12 | 580 | 950 |
| 19 | 454 | 198 |
| 25 | 467 | 370 |

The effect of the dithiane and dithiolium donors with a tricyanovinyl acceptor were compared. A dithiane donor with a $-NO_2$ acceptor is compared to the same donor with a tricyanovinylate acceptor. A thiophene ring is compared to fused thiophene rings with a dithiane donor and a $-NO_2$ acceptor, as well as with a dithiane donor and a tricyanovinyl acceptor.

The increase in the NLO coefficient with fused ring systems, and with tricyanovinyl acceptors is evident. An increase in NLO properties with dithiolium donors is also evident. These compounds also possess the thermal stability and photochemical stability required of NLO materials.

The NLO compounds of the present invention thus possess a combination of NLO properties and thermal and photochemical stability heretofore unobtained by the prior art. At the same time, the compounds have good solubility, high laser damage thresholds, are easily synthesized and have well-known and understood chemical properties. The fused ring and non-fused ring structures of the present invention containing five-membered heteroaromatic rings represent a versatile family of compounds that can be readily varied to increase their second order NLO properties.

The foregoing examples and description of the preferred embodiment should be taken as illustrating, rather than as limiting, the present invention as defined by the claims. As will be readily appreciated, numerous variations and combinations of the features set forth above can be utilized without departing from the present invention as set forth in the claims. Such variations are not regarded as a departure from the spirit and scope of the invention, and all such modifications are intended to be included within the scope of the following claims.

## Claims

**1.** A nonlinear optical compound of the formula:

D-R-A

wherein R is a fused ring system consisting of two or three aromatic or heteroaromatic rings, at least one of which is a five-membered heteroaromatic ring having the structure:

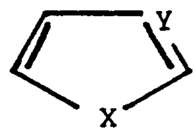

wherein Y is C or N and X is selected from the group consisting of O, S, Se and N; provided:

a) that when R consists of two rings, one of which is benzene, Y is C; and

b) that X is selected from the group consisting of O, S and Se when said ring system comprises:

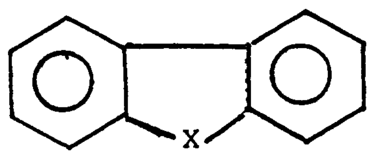

D is an electron donating group, A is an electron accepting group, and D and A are attached to ring members of different rings so that D, A and R form a delocalized resonance configuration.

**2.** The nonlinear optical compound of claim 1, wherein R is a fused ring system consisting of two or three five-membered heteroaromatic rings.

**3.** The nonlinear optical compound of claim 1, wherein R is a fused ring system consisting of one five-membered heteroaromatic ring and one benzene or pyridine ring

**4.** The nonlinear optical compound of claim 1, wherein R is a fused ring system consisting of one five-membered heteroaromatic ring and two rings independently selected from the group consisting of benzene and pyridine rings.

**5.** The nonlinear optical compound of claim 1, wherein R is a fused ring system consisting of two five-membered heteroaromatic rings and a benzene or pyridine ring.

**6.** The compound of claim 1, wherein said electron donating group is selected from the group consisting of $-NR_5R_6$, $-OR_8$, $-SR_8$, $-TeR_8$, $-SeR_8$, $-CH=NR_4$, $-CH=N-NH_2$, $-CH=N-N(R_5R_6)$ and $-CH=C[N(R_5R_6)-]_2$ wherein $R_4$ is hydrogen or an alkyl group containing up to ten carbon atoms, $R_8$ is an alkyl group containing up to six carbon atoms and $R_5$ and $R_6$ are independently selected from the group consisting of hydrogen, alkyl groups containing up to 12 carbon atoms and alkyl groups containing up to 12 carbon atoms having reactive functional groups selected from the group consisting of hydroxyl, ethylene, acetylene, amine, thiol, sulfonic acid and carboxylic acid, or $R_5$ and $R_6$ together form a cyclic group containing up to 8 carbon atoms.

**7.** The compound of claim 6, wherein $R_8$ is a methyl group, $R_4$ is hydrogen or a methyl group and $R_5$ and $R_6$ are independently selected from the group consisting of methyl, ethyl and hexyl, or $R_5$ and $R_6$ together form a cyclopentyl or cyclohexyl functional group.

**8.** The compound of claim 6, wherein said electron donating group comprises $-CH=N-N(R_5R_6)$ or $-CH=C[N(R_5R_6)]_2$, and $R_5$ and $R_6$ together form a cyclic group selected from the group consisting of pyrrolidino, piperidino, piperazino and morpholino groups.

**9.** The compound of claim 1, wherein said electron donating group is selected from the group consisting of:

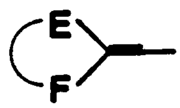

and

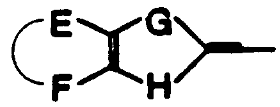

wherein E, F, G and H are members of a saturated or unsaturated five- to eight-membered cyclic ring or two-ring system having five- to eight-membered rings, which are electron donating in nature, and E, F, G and H are heteroatoms independently selected from the group consisting of O, N, S, Se and Te.

**10.** The compound of claim 1, wherein said electron donating group is selected from the group consisting of pyrrolidino, piperidino, piperazino, morpholino, dithiolium and dithiane groups.

**11.** The compound of claim 10, wherein said electron donating group comprises 2-ethylenedithio-1,3-dithiolium.

**12.** The compound of claim 1, further including a second electron donating group attached to the same ring as the electron donating group D so that said delocalized resonance configuration is maintained.

**13.** The compound of claim 1, wherein said electron accepting group is selected from the group consisting of $-NO_2$, -CN, -CHO, $-COR_{10}$, $-COOR_{10}$, $-PO(OR_{10})_2$, $-SO_2R_{10}$, $-SO_3R_{10}$, $-PO(R_{10})_2$ and

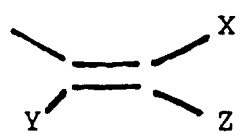

wherein X, Y and Z are independently selected from the group consisting of hydrogen, -CN, $-NO_2$, $-COR_{10}$, $-COOR_{10}$, $-SO_2R_{10}$, $-PO(R_{10})_2$ and $-PO(OR_{10})_2$, wherein $R_{10}$ is an alkyl group containing up to 15 carbon atoms.

**14.** The compound of claim 1, wherein said electron accepting group is selected from the group consisting of $-NO_2$, tricyanoethylene, dicyanoethylene, dinitroethylene, nitrocyanoethylene, nitroesterethylene, N,N-dialkylbarbituric acids, N,N-dialkylthiobarbituric acids, indane-1,3-dione, 3-dicyanoindane-1-one, 1,3-bis-dicyanovinylindane, 3-dicyanovinylindane-1-sulfone and 1,3-bissulfonylindane.

**15.** The compound of claim 14, wherein said electron accepting group comprises tricyanoethylene.

**16.** The compound of claim 1, wherein said electron accepting group comprises

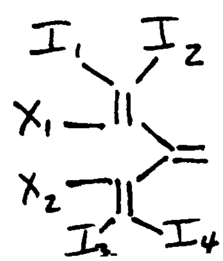

wherein $I_1$, $I_2$, $I_3$ and $I_4$ are independently selected from the group consisting of cyano, nitro, ester, sulfonyl and phosphonyl, and $X_1$ and $X_2$ form a saturated or unsaturated five- to eight-membered cyclic ring or two-ring system having five- to eight-membered rings.

**17.** The compound of claim 1, further including a second electron accepting group attached to the same ring as the electron accepting group A, so that said delocalized resonance configuration is maintained.

18. The compound of claim 2, wherein said fused ring system consists of three five-membered heteroaromatic rings.

19. A nonlinear optical compound of the formula:

D-R-A

wherein R is selected from the group consisting of heteroaromatic ring compounds represented by the structure:

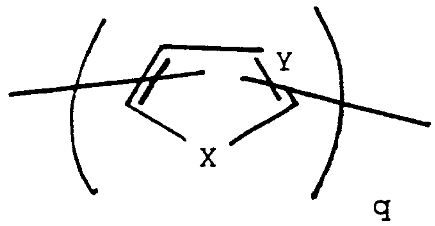

wherein q is an integer between one and four, inclusive, Y is C or N and X is selected from the group consisting of O, S, Se and N;

D is an electron donating group, A is an electron accepting group, and D, A and the one to four rings of R form a delocalized resonance configuration; and

either D is a strong electron donating group, A is a strong electron accepting group, or both D and A are strong electron donating and accepting groups, respectively, wherein

said strong electron donating groups are selected from the group consisting of $-N(CH_3)_2$, pyrrolidino, piperidino, piperazino, morpholino, dithianes, dithioliums,

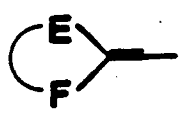 and 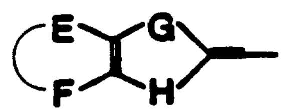

wherein E, F, G and H are members of a saturated or unsaturated five- to eight-membered cyclic ring or two-ring system having five- to eight-membered rings that are electron donating in nature and E, F, G and H are heteroatoms independently selected from the group consisting of O, N, S, Se and Te; and

said strong electron accepting groups are selected from the group consisting of tricyanoethylene, dicyanoethylene, $-NO_2$, dinitroethylene, nitroesterethylene, nitrocyanoethylene, N,N-dialkylbarbituric acids, N,N-dialkylthiobarbituric acids, 3-dicyanovinylindane-1-sulfone, 1,3-bissulfonyl-indane, indane-1,3-dione, 3-dicyanoindane-1-one, 1,3-bisdicyanovinylindane and

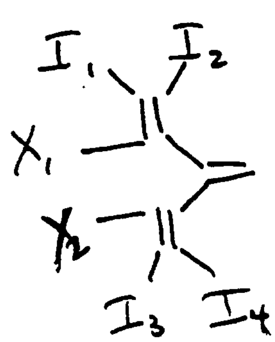

wherein $I_1$, $I_2$, $I_3$ and $I_4$ are independently selected from cyano, nitro, ester, sulfonyl and phosphonyl groups, and $X_1$ and $X_2$ form a saturated or unsaturated five- to eight-membered cyclic ring or two-ring system having five- to eight-membered rings.

**20.** The compound of claim 19, wherein said electron donating group comprises a strong electron donating group and said electron accepting group is selected from the group consisting of -CN, -CHO, $-COR_{10}$, $-COOR_{10}$, $-PO(OR_{10})_2$, $-SO_2R_{10}$, $-SO_3R_{10}$, $-PO(R_{10})_2$ and

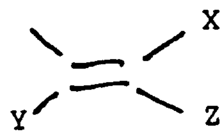

wherein X, Y and Z are independently selected from the group consisting of hydrogen, -CN, $-NO_2$, $-COR_{10}$, $-COOR_{10}$, $-SO_2R_{10}$, $-PO(R_{10})_2$ and $-PO(OR_{10})_2$, wherein $R_{10}$ is an alkyl group containing up to 15 carbon atoms.

**21.** The compound of claim 19, wherein said electron accepting group comprises a strong electron accepting group and said electron donating group is selected from the group consisting of $-NR_5R_6$, $-OR_8$, $-SR_8$, $-TeR_8$, $-SeR_8$, $-CH=NR_4$, $-CH=N-NH_2$, $-CH=N-N(R_5R_6)$ and $-CH=C[N(R_5R_6)]_2$, wherein $R_4$ is hydrogen or an alkyl group containing up to ten carbon atoms, $R_8$ is an alkyl group containing up to six carbon atoms and $R_5$ and $R_6$ are independently selected from the group consisting of hydrogen, alkyl groups containing up to 12 carbon atoms and alkyl groups containing up to 12 carbon atoms having reactive functional groups selected from the group consisting of hydroxyl, ethylene, acetylene, amine, thiol, sulfonic acid and carboxylic acid, or $R_5$ and $R_6$ together form a cyclic group containing up to 8 carbon atoms.

**22.** The compound of claim 19, wherein q is an integer between two and four, inclusive, and D and A are attached to different heteroaromatic rings.

**23.** The compound of claim 22, wherein said electron donating group is a first electron donating group and said compound further includes a second electron donating group attached to the same ring as said first electron donating group so that said delocalized resonance configuration is maintained.

**24.** The compound of claim 22, wherein said electron accepting group is a first electron accepting group and said compound further includes a second electron accepting group attached to the same ring as said first electron accepting group so that said delocalized resonance configuration is maintained.

**25.** A combination exhibiting second order nonlinear optical properties comprising a chemically inert medium and the nonlinear optical compound of claims 1 or 19.

**26.** The combination of claim 22, wherein said nonlinear optical compound has an external field-induced molecular alignment.

**27.** A combination exhibiting second order nonlinear optical properties comprising a polymeric material to which pendant side chains of a nonlinear optical compound are covalently bonded, which combination corresponds to the formula:

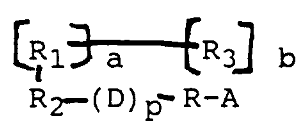

wherein $R_1$ and $R_3$ are monomeric subunits independently selected from the group consisting of acrylate, imide, amide, acrylamide, styrene, vinyl halide, acrylonitrile, vinyl alcohol, vinyl acetate, polyester, polyphenylene ether, polyether ketone, polyether etherketone, acid anhydride, ethylene, propylene, isobutylene, isoprene and polycarbonate monomeric subunits, wherein each $R_1$ subunit includes a functional group through which said side chains may be attached;

p is zero or one;

$R_2$ is a straight-chained or branched alkyl, alkoxy, thioalkyl or aminoalkyl group containing from one

to ten carbon atoms attached to said monomeric subunit at said functional group, with the proviso that when p is zero, $R_2$ is attached to said functional group by an alkyl moiety and $R_2$ is an alkoxy, alkylthio or alkyl amino group;

the ratio of a to b is between about 1:99 and about 50:50;

R is selected from the group consisting of fused ring systems consisting of two or three aromatic or heteroaromatic rings, at least one of which is a five-membered heteroaromatic ring having the structure:

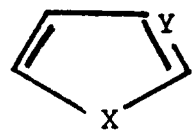

wherein Y is C or N and X is selected from the group consisting of O, S, Se and N;

provided:

a) that when R consists of two rings, one of which is benzene, Y is C; and

b) that X is selected from the group consisting of O, S and Se when said ring system comprises:

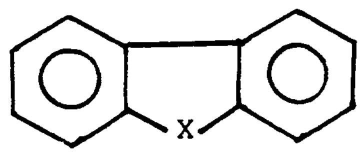

D, when present, is a first electron donating group, A is a first electron accepting group, and D or $R_2$ and A are attached to ring members of different rings so that D or $R_2$, A and R form a delocalized resonance configuration.

**28.** A combination exhibiting second order nonlinear optical properties comprising a polymeric material to which pendant side chains of a nonlinear optical compound are covalently bonded, which combination corresponds to the formula:

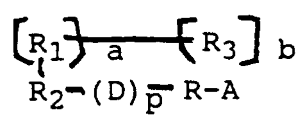

wherein $R_1$ and $R_3$ are monomeric subunits independently selected from the group consisting of acrylate, imide, amide, acrylamide, styrene, vinyl halide, acrylonitrile, vinyl alcohol, vinyl acetate, polyester, polyphenylene ether, polyether ketone, polyether etherketone, acid anhydride, ethylene, propylene, isobutylene, isoprene and polycarbonate monomeric subunits, wherein each $R_1$ subunit includes a functional group through which said side chains may be attached;

p is zero or one;

$R_2$ is a straight-chained or branched alkyl, alkoxy, thioalkyl or aminoalkyl group containing from one to ten carbon atoms attached to said monomeric subunit at said functional group, with the proviso that when p is zero, $R_2$ is attached to said functional group by an alkyl moiety and $R_2$ is an alkoxy, alkylthio or alkyl amino group;

the ratio of a to b is between about 1:99 and about 50:50;

R is selected from the group consisting of heteroaromatic compounds represented by the structure:

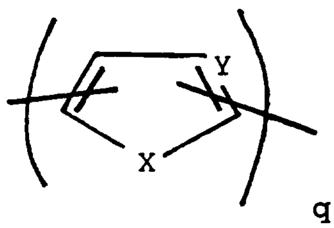

wherein q is an integer, between one and four, inclusive, Y is C or N and X is selected from the group consisting of O, S, Se and N;

D is an electron donating group, A is an electron accepting group, and D, A and the one to four rings of R form a delocalized resonance configuration; and

either D is a strong electron donating group, A is a strong electron accepting group, or both D and A are strong electron donating and accepting groups, respectively, wherein

said strong electron donating groups are selected from the group consisting of $-N(CH_3)_2$, pyrrolidino, piperidino, piperazino, morpholino, dithianes, dithioliums,

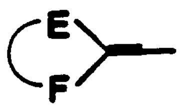

and

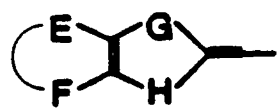

wherein E, F, G and H are members of a saturated or unsaturated five- to eight-membered cyclic ring or two-ring system having five- to eight-membered rings that are electron donating in nature and E, F, G and H are heteroatoms independently selected from the group consisting of O, N, S, Se and Te; and

said strong electron accepting groups are selected from the group consisting of tricyanoethylene, dicyanoethylene, $-NO_2$, dinitroethylene, nitroesterethylene, nitrocyanoethylene, N,N-dialkylbarbituric acids, 3-dicyanovinylindane-1-sulfone, 1,3-bissulfonylindane, indane-1,3-dione, 3-dicyanoindane-1-one, 1,3-bisdicyanovinylindane and

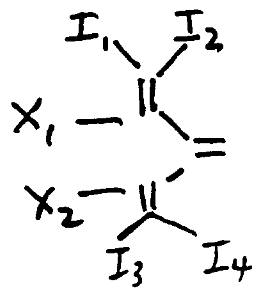

wherein $I_1$, $I_2$, $I_3$ and $I_4$ are independently selected from cyano, nitro, ester, sulfonyl and phosphonyl groups, and $X_1$ and $X_2$ form a saturated or unsaturated five- to eight-membered cyclic ring or two-ring system having five- to eight-membered rings.

**29.** The combination of claim 27 or claim 28, wherein $R_2$ is selected from the group consisting of $(-CH_2-)_x$, $(-CH_2-R_9-)_x$, $(-CH_2-CH_2-R_9-)_{x/2}$ and $(-CH_2-CH_2-CH_2-R_9-)_{x/3}$ wherein $R_9$ is selected from the group consisting of O, S and NH, and X is between one and ten, with the proviso that for $(-CH_2-CH_2-R_9-)$, X is a multiple of two and for $(-CH_2-CH_2-CH_2-R_9-)$, X is a multiple of three.

**30.** The combination of claim 27 or claim 28, wherein $R_2$ contains from four to six carbon atoms.

**31.** The combination of claim 27 or claim 28, wherein the ratio of a to b is between about 5:95 and about 40:60.

**32.** The combination of claim 31, wherein the ratio of a to b is about 25:75.

**33.** The combination of claim 27 or claim 28, wherein $R_1$ and $R_3$ are independently selected from the group consisting of imide, acrylate, alkyl-branched acrylate, acrylamide, alkyl-branched acrylamide, styrene, alpha-alkyl styrene, vinyl acetate, polyether ketone, polyether etherketone and ethylene monomeric subunits.

**34.** The combination of claim 33, wherein $R_1$ and $R_3$ are independently selected from the group consisting of imide, acrylate, methacrylate, polyether ketone and polyether etherketone monomeric subunits.

**35.** A method of preparing a polymer having second order nonlinear optical properties, which method comprises the steps of:

reacting a base polymer having pendant side chains, said base polymer comprising recurring structural units represented by the formula:

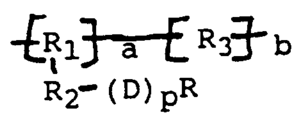

with tetracyanoethylene in a basic solvent at a temperature between about 0 °C and about 150 °C, so that R of said pendant side chains is tricyanovinylated; and

recovering the resulting polymer having pendant tricyanovinylated side chains;

wherein $R_1$ and $R_3$ are monomeric subunits independently selected from the group consisting of acrylate, imide, amide, acrylamide, styrene, vinyl halide, acrylonitrile, vinyl alcohol, vinyl acetate, polyester, polyphenylene ether, polyether ketone, polyether etherketone, acid anhydride, ethylene, propylene, isobutylene, isoprene and polycarbonate monomeric subunits, wherein each $R_1$ subunit includes a functional group through which said side chains may be attached;

p is zero or one;

$R_2$ is a straight-chained or branched alkyl, alkoxy, thioalkyl or aminoalkyl group containing from one to ten carbon atoms attached to said monomeric subunit at said functional group, with the proviso that when p is zero, $R_2$ is attached to said functional group by an alkyl moiety and $R_2$ is an alkoxy, alkylthio or alkyl amino group;

the ratio of a to b is between about 1:99 and about 50:50;

R is selected from the group consisting of:

a) fused ring systems consisting of two or three aromatic or heteroaromatic rings, at least one of which is a five-membered heteroaromatic ring having the structure:

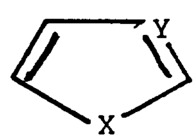

wherein Y is C or N and X is selected from the group consisting of O, S, Se and N;

provided:

i) that when R consists of two rings, one of which is benzene, Y is C; and

ii) that X is selected from the group consisting of O, S and Se when said ring system comprises;

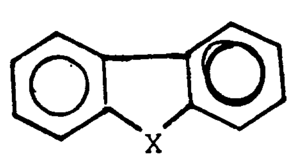

and, b) heteroaromatic ring compounds represented by the structure:

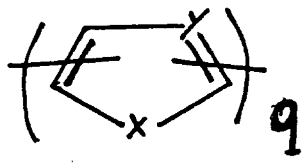

wherein q is an integer between one and four, inclusive, Y is C or N and X is selected from the group consisting of O, S, Se and N;

D, when present, is an electron donating group, D or $R_2$ are attached to a ring different from the ring to be tricyanovinylated, and D or $R_2$ are attached to R so that a delocalized resonance configuration is formed when R is tricyanovinylated.

**36.** The method of claim 35, wherein said basic solvent is selected from the group consisting of dimethylformamide (DMF), pyridine, N,N'-dimethyl-acetamide, N-methylpyrrolidone and tertiary amines.

**37.** The method of claim 36, wherein said solvent is DMF.

**38.** The method of claim 35, wherein the temperature of said reacting step is about 100 ° C.

**39.** The method of claim 35, wherein $R_2$ is selected from the group consisting of $(-CH_2-)_x$,$(-CH_2-R_9-)_x$, $(-CH_2-CH_2-R_9-)_{x/2}$ and $(-CH_2-CH_2-CH_2-R_9-)_{x/3}$ wherein $R_9$ is selected from the group consisting of O, S and NH, and X is between one and ten, with the proviso that for $(-CH_2-CH_2-R_9-)$, X is a multiple of two and for $(-CH_2-CH_2-CH_2-R_9-)$, X is a multiple of three.

**40.** The method of claim 35, wherein $R_2$ is an alkylamino group.

**41.** The method of claim 35, wherein $R_2$ contains six carbon atoms.

**42.** The method of claim 35, wherein $R_1$ and $R_3$ are independently selected from the group consisting of acrylate, alkyl-branched acrylate, acrylamide, alkyl-branched acrylamide, imide, styrene, alpha-alkyl styrene, vinyl acetate, polyether ketone, polyether etherketone and ethylene monomeric subunits.

**43.** The method of claim 42, wherein $R_1$ and $R_3$ are independently selected from the group consisting of acrylate, imide, methacrylate, polyether ketone and polyether etherketone monomeric subunits.

**44.** The method of claim 35, wherein the ratio of a to b is between about 5:95 and about 40:60.

**45.** The method of claim 44, wherein the ratio of a to b is about 25:75.

**46.** A base polymer having pendant side chains capable of being tricyanovinylated to form a polymer having second order nonlinear optical properties, said base polymer comprising recurring structural units represented by the formula:

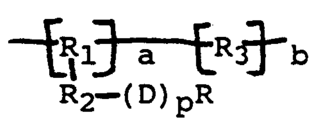

wherein $R_1$ and $R_3$ are monomeric subunits independently selected from the group consisting of acrylate, imide, amide, acrylamide, styrene, vinyl halide, acrylonitrile, vinyl alcohol, vinyl acetate, polyester, polyphenylene ether, polyether ketone, polyether etherketone, acid anhydride, ethylene, propylene, isobutylene, isoprene and polycarbonate monomeric subunits, wherein each $R_1$ subunit includes a functional group through which said side chains may be attached;

p is zero or one;

$R_2$ is a straight-chained or branched alkyl, alkoxy, thioalkyl or aminoalkyl group containing from one to ten carbon atoms attached to said monomeric subunit at said functional group, with the proviso that when p is zero, $R_2$ is attached to said functional group by an alkyl moiety and $R_2$ is an alkoxy, alkylthio

or alkyl amino group;

the ratio of a to b is between about 1:99 and about 50:50;

R is selected from the group consisting of:

a) fused ring systems consisting of two or three aromatic or heteroaromatic rings, at least one of which is a five-membered heteroaromatic ring having the structure:

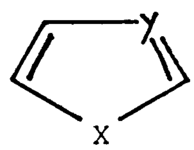

wherein Y is C or N and X is selected from the group consisting of O, S, Se and N;

provided:

i) that when R consists of two rings, one of which is benzene, Y is C; and

ii) that X is selected from the group consisting of O, S and Se when said ring system comprises;

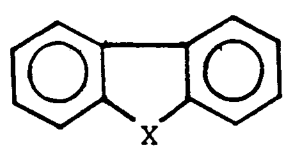

and, b) heteroaromatic ring compounds represented by the structure:

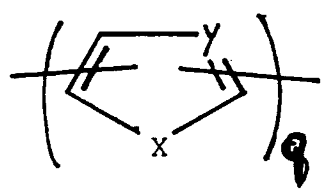

wherein q is an integer between one and four, inclusive, Y is C or N and X is selected from the group consisting of O, S, Se and N;

D, when present, is an electron donating group, D or $R_2$ are attached to a ring different from the ring to be tricyanovinylated, and D or $R_2$ are attached to R so that a delocalised resonance configuration is formed when R is tricyanovinylated.

**47.** The polymer of claim 46, wherein $R_2$ is selected from the group consisting of $(-CH_2-)_x$,$(-CH_2-R_9-)_x$, $(-CH_2-CH_2-R_9-)_{x/2}$ and $(-CH_2-CH_2-CH_2-R_9-)_{x/3}$ wherein $R_9$ is selected from the group consisting of O, S and NH, and X is between one and ten, with the proviso that for $(-CH_2-CH_2-R_9-)$, X is a multiple of two and for $(-CH_2-CH_2-CH_2-R_9-)$, X is a multiple of three.

**48.** The polymer of claim 46, wherein $R_2$ is an alkylamino group.

**49.** The polymer of claim 46, wherein $R_2$ contains six carbon atoms.

**50.** The polymer of claim 46, wherein $R_1$ and $R_3$ are independently selected from the group consisting of acrylate, alkyl-branched acrylate, acrylamide, alkyl-branched acrylamide, styrene, alpha-alkyl styrene, vinyl acetate, polyether ketone, polyether etherketone and ethylene monomeric subunits.

**51.** The polymer of claim 50, wherein $R_1$ and $R_3$ are independently selected from the group consisting of acrylate, methacrylate, polyether ketone and polyether etherketone monomeric subunits.

**52.** The polymer of claim 46, wherein said ratio of a to b is between about 5:95 and about 40:60.

**53.** The polymer of claim 52, wherein said ratio of a to b is about 25:75.

European Patent Office

# EUROPEAN SEARCH REPORT

Application Number

EP 93 20 2330
PAGE1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A<br><br>D | EP-A-0 384 811 (RHONE-POULENC CHIMIE)<br>* the whole document *<br>& CA-A-2 010 528<br>--- | 1,25,27 | C07D495/14<br>C07D333/54<br>C07D333/76<br>C07D307/91<br>C07D495/04<br>G02F1/35 |
| A,D | EP-A-0 353 606 (KONICA CORPORATION)<br>* the whole document *<br>--- | 1,25,27 | |
| A,D | US-A-3 978 029 (WILLIAM W. LIMBURG)<br>* the whole document *<br>--- | 1,25,27 | |
| A,D | SYNTHETIC METALS<br>vol. 41-43, 1991, THE NETHERLANDS<br>page 2393<br>A. BERLIN 'Monomer tailoring to control the redox potentials of conductive Polyheterocycles'<br>* page 2393 *<br>--- | 1 | |
| A,D | CANADIAN JOURNAL OF CHEMISTRY<br>vol. 56, no. 9, 1 May 1978, CANADA<br>pages 1429 - 1434<br>SAMRETH SOTH 'Recherches en série hétérocyclique. XXIX'<br>* the whole document *<br>--- | 1 | **TECHNICAL FIELDS SEARCHED (Int. Cl.5 )**<br>C07D<br>G02F |
| A,D | THE JOURNAL OF ORGANIC CHEMISTRY<br>vol. 36, no. 14, 16 July 1971, COLUMBUS OHIO<br>pages 1998 - 2000<br>FEIKE DE JONG 'Synthesis of Dithienothiophenes'<br>* the whole document *<br>---<br>-/-- | 1 | |

The present search report has been drawn up for all claims

| Place of search | Date of completion of the search | Examiner |
|---|---|---|
| BERLIN | 15 DECEMBER 1993 | KYRIAKAKOU G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 93 20 2330
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A,D | THE JOURNAL OF ORGANIC CHEMISTRY<br>vol. 36, no. 12, 18 June 1971, COLUMBUS OHIO<br>pages 1645 - 1648<br>FEIKE DE JONG 'The synthesis, Oxidation and electronic Spectra of four Dithienothiophenes'<br>* the whole document *<br>--- | 1 | |
| A,D | DIE MAKROMOLEKULARE CHEMIE<br>vol. 181, no. 10, 1 October 1980, BASEL<br>pages 2199 - 2206<br>SHIGEO TAZUKE ET AL 'Donor Acceptor Interactions in Polymeric Systems, 5.'<br>* the whole document *<br>--- | 1,25,27 | |
| A,D | ANGEW. CHEM. INT. ED. ENGL.<br>vol. 23, 1984, WEINHEIM<br>pages 690 - 703<br>DAVID J. WILLIAMS 'Organic Polymeric and non-Polymeric Materials with large Optical nonlinearities'<br>* the whole document *<br>--- | 1,25,27 | TECHNICAL FIELDS SEARCHED (Int. Cl.5 ) |
| A,D | JOURNAL OF THE OPTICAL SOCIETY OF AMERICA B, OPTICAL PHYSICS<br>vol. 6, no. 7, July 1989,<br>pages 1339 - 1350<br>K. D. SINGER ET AL. 'Second-order nonlinear-optical properties of donor- and acceptor-substituted aromatic compounds.'<br>* the whole document *<br>----- | 1,25,27 | |

The present search report has been drawn up for all claims

| Place of search | Date of completion of the search | Examiner |
|---|---|---|
| BERLIN | 15 DECEMBER 1993 | KYRIAKAKOU G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document